# FASCICULE DE BREVET EUROPEEN

(11) **EP 0 820 448 B1**
(45) Date de publication et mention de la délivrance du brevet: **31.05.2000**
(21) Numéro de dépôt: 96913578.9
(22) Date de dépôt: 12.04.1996
(51) Int. Cl.: C07D 305/14, C07D 407/12, C07D 409/12, C07D 417/12, A61K 31/335

(54) **NOUVEAUX TAXOIDES, LEUR PREPARATION ET LES COMPOSITIONS PHARMACEUTIQUES QUI LES CONTIENNENT**
TAXAL DERIVATE, DEREN HERSTELLUNG UND DIESE ENTHALTENDE PHARMAZEUTISCHE ZUSAMMENSTELLUNGEN
NOVEL TAXOIDS, PREPARATION THEREOF, AND PHARMACEUTICAL COMPOSITIONS CONTAINING SAME

(30) Priorité: 14.04.1995 FR 9504559
(43) Date de publication de la demande: 28.01.1998
(73) Titulaire: Aventis Pharma S.A., 92160 Antony (FR)
(72) Inventeur: Bouchard, Hervé, F-94320 Thiais (FR); Commerçon, Alain, F-94400 Vitry-sur-Seine (FR)
(74) Mandataire: Le Pennec, Magali
(86) Numéro de dépôt international: FR9600559
(87) Numéro de publication internationale: WO9632387

(56) Documents cités:
- WO-A-95/09163
- US-A- 5 254 580

## Description

La présente invention concerne de nouveaux taxoïdes de formule générale : 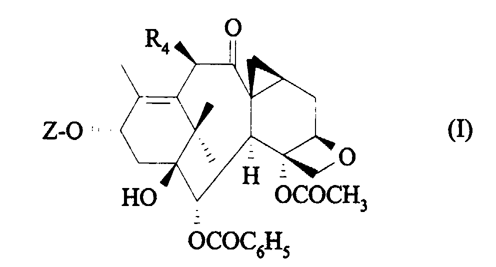 dans laquelle :
Z représente un atome d'hydrogène ou un radical de formule générale : 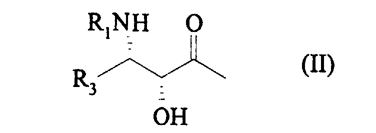 dans laquelle :
R₁ représente un radical benzoyle éventuellement substitué par un ou plusieurs atomes ou radicaux, identiques ou différents, choisis parmi les atomes d'halogène et les radicaux alcoyles contenant 1 à 4 atomes de carbone, alcoxy contenant 1 à 4 atomes de carbone ou trifluorométhyle, thénoyle ou furoyle ou un radical R₂-O-CO- dans lequel R₂ représente :
- un radical alcoyle contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone, bicycloalcoyle contenant 7 à 10 atomes de carbone, ces radicaux étant éventuellement substitués par un ou plusieurs substituants choisis parmi les atomes d'halogène et les radicaux hydroxy, alcoxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle (éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone), cyano, carboxy ou alcoxycarbonyle dont la partie alcoyle contient 1 à 4 atomes de carbone,
- un radical phényle ou α- ou β-naphtyle éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone ou un radical hétérocyclique aromatique à 5 chaînons choisi de préférence parmi les radicaux furyle et thiényle,
- ou un radical hétérocyclyle saturé contenant 4 à 6 atomes de carbone éventuellement substitué par un ou plusieurs radicaux alcoyles contenant 1 à 4 atomes de carbone,
   R₃ représente un radical
   - alcoyle droit ou ramifié contenant 1 à 8 atomes de carbone,
   - alcényle droit ou ramifié contenant 2 à 8 atomes de carbone,
   - alcynyle droit ou ramifié contenant 2 à 8 atomes de carbone,
   - cycloalcoyle contenant 3 à 6 atomes de carbone,
   - phényle ou α- ou β-naphtyle
   éventuellement substitué par un ou plusieurs atomes ou radicaux choisis parmi les atomes d'halogène et les radicaux alcoyles, alcényles, alcynyles, aryles, aralcoyles, alcoxy, alcoylthio, aryloxy, arylthio, hydroxy, hydroxyalcoyle, mercapto, formyle, acyle, acylamino, aroylamino, alcoxycarbonyl-amino, amino, alcoylamino, dialcoylamino, carboxy, alcoxycarbonyle, carbamoyle, alcoylcarbamoyle, dialcoylcarbamoyle, cyano, nitro et trifluorométhyle,
   - ou un hétérocycle aromatique ayant 5 chaînons et contenant un ou plusieurs hétéroatomes, identiques ou différents, choisis parmi les atomes d'azote, d'oxygène ou de soufre et éventuellement substitué par un ou plusieurs substituants, identiques ou différents, choisis parmi les atomes d'halogène et les radicaux alcoyles, aryles, amino, alcoylamino, dialcoylamino, alcoxycarbonylamino, acyle, arylcarbonyle, cyano, carboxy, carbamoyle, alcoylcarbamoyle, dialcoylcarbamoyle ou alcoxycarbonyle, étant entendu que, dans les substituants des radicaux phényle, α- ou β-naphtyle et hétérocyclyles aromatiques, les radicaux alcoyles et les portions alcoyles des autres radicaux contiennent 1 à 4 atomes de carbone et que les radicaux alcényles et alcynyles contiennent 2 à 8 atomes de carbone et que les radicaux aryles sont des radicaux phényles ou α- ou β-naphtyles,
   R₄ représente un radical
   - alcoxy contenant 1 à 6 atomes de carbone en chaîne droite ou ramifiée,
   - alcényloxy contenant 3 à 6 atomes de carbone en chaîne droite ou ramifiée,
   - alcynyloxy contenant 3 à 6 atomes de carbone en chaîne droite ou ramifiée,
   - cycloalcoyloxy contenant 3 à 6 atomes de carbone,
   - cycloalcényloxy contenant 3 à 6 atomes de carbone
éventuellement substitué par un ou plusieurs atomes d'halogène ou par un radical alcoxy contenant 1 à 4 atomes de carbone, alcoylthio contenant 1 à 4 atomes de carbone, ou un radical carboxy, alcoyloxycarbonyle dont la partie alcoyle contient 1 à 4 atomes de carbone, cyano, carbamoyle, N-alcoylcarbamoyle ou N,N-dialcoylcarbamoyle dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote éventuellement substitué par un radical alcoyle contenant 1 à 4 atomes de carbone, ou un radical phényle ou un radical phénylalcoyle dont la partie alcoyle contient 1 à 4 atomes de carbone.

De préférence les radicaux aryles pouvant être représentés R₃ sont des radicaux phényles ou α- ou β-naphtyles éventuellement substitués par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, dialcoylcarbamoyle, cyano, nitro et trifluoro-méthyle, étant entendu que les radicaux alcoyles et les portions alcoyles des autres radicaux contiennent 1 à 4 atomes de carbone, que les radicaux alcényles et alcynyles contiennent 2 à 8 atomes de carbone et que les radicaux aryles sont des radicaux phényles ou α- ou β-naphtyles.

De préférence les radicaux hétérocycliques pouvant être représentés par R₃ sont des radicaux hétérocycliques aromatiques ayant 5 chaînons et contenant un ou plusieurs atomes, identiques ou différents, choisis parmi les atomes d'azote, d'oxygène ou de soufre, éventuellement substitués par un ou plusieurs substituants, identiques ou différents, choisis parmi les atomes d'halogène (fluor, chlore, brome, iode) et les radicaux alcoyles contenant 1 à 4 atomes de carbone, aryles contenant 6 à 10 atomes de carbone, alcoxy contenant 1 à 4 atomes de carbone, aryloxy contenant 6 à 10 atomes de carbone, amino, alcoylamino contenant 1 à 4 atomes de carbone, dialcoylamino dont chaque partie alcoyle contient 1 à 4 atomes de carbone, acylamino dont la partie acyle contient 1 à 4 atomes de carbone, alcoxycarbonylamino contenant 1 à 4 atomes de carbone, acyle contenant 1 à 4 atomes de carbone, arylcarbonyle dont la partie aryle contient 6 à 10 atomes de carbone, cyano, carboxy, carbamoyle, alcoylcarbamoyle dont la partie alcoyle contient 1 à 4 atomes de carbone, dialcoylcarbamoyle dont chaque partie alcoyle contient 1 à 4 atomes de carbone ou alcoxycarbonyle dont la partie alcoxy contient 1 à 4 atomes de carbone.

De préférence le radical R₄ représente un radical alcoxy droit ou ramifié contenant 1 à 6 atomes de carbone éventuellement substitué par un radical méthoxy, éthoxy, méthyltio, éthylthio, carboxy, méthoxycarbonyle, éthoxycarbonyle, cyano, carbamoyle, N-méthyl-carbamoyle, N-éthylcarbamoyle, N,N-diméthylcarbamoyle, N,N-diéthylcarbamoyle, N-pyrrolidinocarbonyle, N-pipéridinocarbonyle ou phényle.

Plus particulièrement, la présente invention concerne les produits de formule générale (I) dans laquelle Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical alcoyle contenant 1 à 6 atomes de carbone, alcényle contenant 2 à 6 atomes de carbone, cycloalcoyle contenant 3 à 6 atomes de carbone, phényle éventuellement substitué par un ou plusieurs atomes ou radicaux, identiques ou différents choisis parmi les atomes d'halogène (fluor, chlore) et les radicaux alcoyles (méthyle), alcoxy (méthoxy), dialcoylamino (diméthylamino), acylamino (acétylamino), alcoxycarbonylamino (tert-butoxycarbonylamino) ou trifluorométhyle ou un radical furyle-2 ou -3, thiényle-2 ou -3 ou thiazolyle-2, -4 ou -5 et R₄ représente un radical alcoyloxy droit ou ramifié contenant 1 à 6 atomes de carbone.

Plus particulièrement encore, la présente invention concerne les produits de formule générale (I) dans laquelle Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical isobutyle, isobutényle, butényle, cyclohexyle, phényle, furyle-2, furyle-3, thiényle-2, thiényle-3, thiazolyle-2, thiazolyle-4 ou thiazolyle-5, R₄ représente un radical méthoxy ou éthoxy.

Les composés de la présente demande se différencient de ceux décrits dans les documents WO 95/09163 et US 5,254,580 par la présence en position 10 d'une fonction éther au lieu d'une fonction alcool.

Les produits de formule générale (I) dans laquelle Z représente un radical de formule générale (II) présentent des propriétés antitumorales et antileucémiques remarquables.

Selon la présente invention, les nouveaux produits de formule générale (I) dans laquelle Z représente un radical de formule générale (II) peuvent être obtenus par estérification d'un produit de formule générale : 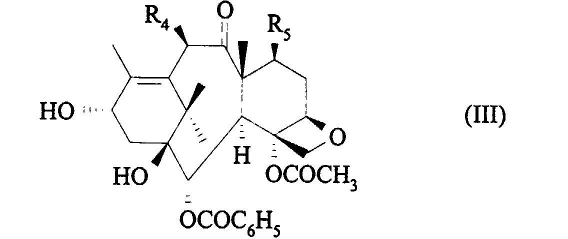 dans laquelle R₄ est défini comme précédemment et R₅ représente un radical trifluorométhanesulfonyloxy ou forme une liaison avec l'atome de carbone du radical méthyle en α de façon à former un cycle cyclopropane, au moyen d'un acide de formule générale : 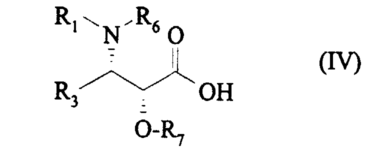 dans laquelle R₁ et R₃ sont définis comme précédemment, ou bien R₆ représente un atome d'hydrogène et R₇ représente un groupement protecteur de la fonction hydroxy, et ou bien R₆ et R₇ forment ensemble un hétérocycle saturé à 5 ou 6 chaînons, ou d'un dérivé de cet acide pour obtenir un ester de formule générale : 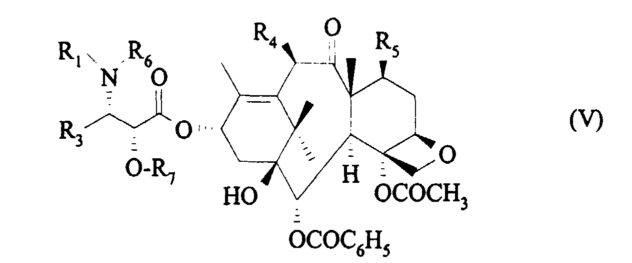 dans laquelle R₁, R₃, R₄, R₅, R₆ et R₇ sont définis comme précédemment, suivi du remplacement des groupements protecteurs représentés par R₇ et/ou R₆ et R₇ par des atomes d'hydrogène et, lorsque R₅ représente un radical trifluorométhanesulfonyloxy, de l'élimination de ce radical de façon à former un cycle cyclopropane avec l'atome de carbone du radical méthyle en α.

L'estérification au moyen d'un acide de formule générale (IV) peut être effectuée en présence d'un agent de condensation (carbodiimide, carbonate réactif) et d'un agent d'activation (aminopyridines) dans un solvant organique (éther, ester, cétones, nitriles, hydrocarbures aliphatiques, hydrocarbures aliphatiques halogénés, hydrocarbures aromatiques) à une température comprise entre -10 et 90°C.

L'estérification peut aussi être réalisée en utilisant l'acide de formule générale (IV) sous forme d'anhydride symétrique en opérant en présence d'un agent d'activation (aminopyridines) dans un solvant organique (éthers, esters, cétones, nitriles, hydrocarbures aliphatiques, hydrocarbures aliphatiques halogénés, hydrocarbures aromatiques) à une température comprise entre 0 et 90°C.

L'estérification peut aussi être réalisée en utilisant l'acide de formule générale (IV) sous forme d'halogénure ou sous forme d'anhydride mixte avec un acide aliphatique ou aromatique, éventuellement préparé in situ, en présence d'une base (amine aliphatique tertiaire) en opérant dans un solvant organique (éthers, esters, cétones, nitriles, hydrocarbures aliphatiques, hydrocarbures aliphatiques halogénés, hydrocarbures aromatiques) à une température comprise entre 0 et 80°C.

De préférence, R₆ représente un atome d'hydrogène et R₇ représente un groupement protecteur de la fonction hydroxy ou bien R₆ et R₇ forment ensemble un hétérocycle saturé à 5 ou 6 chaînons.

Lorsque R₆ représente un atome d'hydrogène, R₇ représente de préférence un radical méthoxyméthyle, éthoxy-1 éthyle, benzyloxyméthyle, triméthylsilyle, triéthylsilyle, β-triméthylsilyléthoxyméthyle, benzyloxycarbonyle ou tétrahydro-pyrannyle.

Lorsque R₆ et R₇ forment ensemble un hétérocycle, celui-ci est de préférence un cycle oxazolidine éventuellement mono-substitué ou gem-disubstitué en position -2.

Le remplacement des groupements protecteurs R₇ et/ou R₆ et R₇ par des atomes d'hydrogène peut être effectué, selon leur nature de la manière suivante :
1) lorsque R₆ représente un atome d'hydrogène et R₇ représente un groupement protecteur de la fonction hydroxy, le remplacement des groupements protecteurs par des atomes d'hydrogène s'effectue au moyen d'un acide minéral (acide chlorhydrique, acide sulfurique, acide fluorhydrique) ou organique (acide acétique, acide méthane-sulfonique, acide trifluorométhanesulfonique, acide p.toluènesulfonique) utilisé seul ou en mélange en opérant dans un solvant organique choisi parmi les alcools, les éthers, les esters, les hydrocarbures aliphatiques, les hydrocarbures aliphatiques halogénés, les hydrocarbures aromatiques ou les nitriles à une température comprise entre -10 et 60°C,
2) lorsque R₆ et R₇ forment ensemble un hétérocycle saturé à 5 ou 6 chaînons et plus particulièrement un cycle oxazolidine de formule générale : 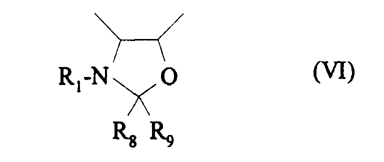 dans laquelle R₁ est défini comme précédemment, R₈ et R₉, identiques ou différents, représentent un atome d'hydrogène ou un radical alcoyle contenant 1 à 4 atomes de carbone, ou un radical aralcoyle dont la partie alcoyle contient 1 à 4 atomes de carbone et la partie aryle représente, de préférence, un radical phényle éventuellement substitué par un ou plusieurs radicaux alcoxy contenant 1 à 4 atomes de carbone, ou un radical aryle représentant, de préférence un radical phényle éventuellement substitué par un ou plusieurs radicaux alcoxy contenant 1 à 4 atomes de carbone, ou bien R₈ représente un radical alcoxy contenant 1 à 4 atomes de carbone ou un radical trihalométhyle tel que trichlorométhyle ou un radical phényle substitué par un radical trihalométhyle tel que trichlorométhyle et R₉ représente un atome d'hydrogène, ou bien R₈ et R₉ forment ensemble avec l'atome de carbone auquel ils sont liés un cycle ayant 4 à 7 chaînons, le remplacement du groupement protecteur formé par R₆ et R₇ par des atomes d'hydrogène peut être effectué, selon les significations de R₁, R₈ et R₉, de la manière suivante :
   a) lorsque R₁ représente un radical tert-butoxycarbonyle, R₈ et R₉, identiques ou différents, représentent un radical alcoyle ou un radical aralcoyle (benzyle) ou aryle (phényle), ou bien R₈ représente un radical trihalométhyle ou un radical phényle substitué par un radical trihalométhyle, et R₉ représente un atome d'hydrogène, ou bien R₈ et R₉ forment ensemble un cycle ayant de 4 à 7 chaînons, le traitement de l'ester de formule générale (V) par un acide minéral ou organique éventuellement dans un solvant organique tel qu'un alcool conduit au produit de formule générale : 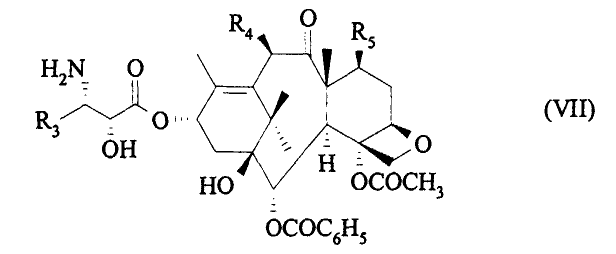 dans laquelle R₃, R₄ et R₅ sont définis comme précédemment, qui est acylé au moyen de chlorure de benzoyle dans lequel le noyau phényle est éventuellement substitué, de chlorure de thénoyle, de chlorure de furoyle ou d'un produit de formule générale :

      R₂-O-CO-X (VIII)

      dans laquelle R₂ est défini comme précédemment et X représente un atome d'halogène (fluor, chlore) ou un reste -O-R₂ ou -O-CO-O-R₂, pour obtenir un produit de formule générale (I) dans laquelle Z représente un radical de formule générale (II).
      De préférence, le produit de formule générale (V) est traité par l'acide formique à une température voisine de 20°C pour fournir le produit de formule générale (VII).
      De préférence, l'acylation du produit de formule générale (VII) au moyen d'un chlorure de benzoyle dans lequel le radical phényle est éventuellement substitué, de chlorure de thénoyle ou de chlorure de furoyle ou d'un produit de formule générale (VIII) est effectuée dans un solvant organique inerte choisi parmi les esters tels que l'acétate d'éthyle, l'acétate d'isopropyle ou l'acétate de n.butyle et les hydrocarbures aliphatiques halogénés tels que le dichlorométhane ou le dichloro-1,2 éthane en présence d'une base minérale telle que le bicarbonate de sodium ou organique telle que la triéthylamine. La réaction est effectuée à une température comprise entre 0 et 50°C, de préférence voisine de 20°C.
   b) lorsque R₁ représente un radical benzoyle éventuellement substitué, thénoyle ou furoyle ou un radical R₂O-CO- dans lequel R₂ est défini comme précédemment, R₈ représente un atome d'hydrogène ou un radical alcoxy contenant 1 à 4 atomes de carbone ou un radical phényle substitué par un ou plusieurs radicaux alcoxy contenant 1 à 4 atomes de carbone et R₉ représente un atome d'hydrogène, le remplacement du groupement protecteur formé par R₆ et R₇ par des atomes d'hydrogène s'effectue en présence d'un acide minéral (acide chlorhydrique, acide sulfurique) ou organique (acide acétique, acide méthanesulfonique, acide trifluorométhanesulfonique, acide p.toluènesulfonique) utilisé seul ou en mélange en quantité stoechiométrique ou catalytique, en opérant dans un solvant organique choisi parmi les alcools, les éthers, les esters, les hydrocarbures aliphatiques, les hydrocarbures aliphatiques halogénés et les hydrocarbures aromatiques à une température comprise entre -10 et 60°C, de préférence entre 15 et 30°C.

Généralement, lorsque R₅ représente un radical trifluorométhanesulfonyloxy, la transformation du produit obtenu après déprotection en dérivé cyclopropané s'effectue au moyen d'un halogénure de métal alcalin (iodure de sodium, fluorure de potassium) ou d'un azoture de métal alcalin (azoture de sodium) ou d'un sel d'ammonium en opérant dans un solvant organique choisi parmi les éthers (tétrahydrofuranne, diisopropyléther, méthyl t.butyléther), les nitriles (acétonitrile) ou les esters aliphatiques (acétate d'éthyle) seul ou en mélange à une température comprise entre 20°C et la température d'ébullition du mélange réactionnel.

Selon l'invention, les produits de formule générale (III) dans laquelle R₄ est défini comme précédemment et R₅ sont définis comme précédemment, peuvent être obtenus à partir de la 10-désacétyl-baccatine III de formule :

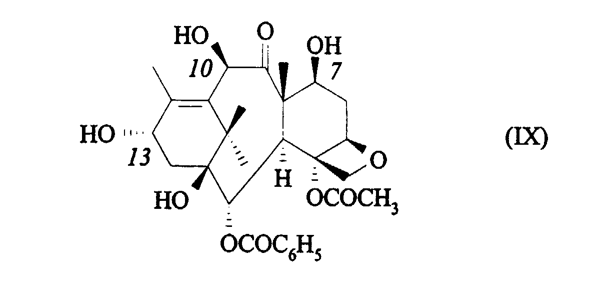

Il peut être particulièrement avantageux de protéger sélectivement les fonctions hydroxy en positions 7 et 13, par exemple sous forme d'un di-éther silylé qui peut être obtenu par action d'un halogénure de silyle de formule générale :

(R')₃-Si-Hal (X)

dans laquelle les symboles R', identiques ou différents, représentent un radical alcoyle contenant 1 à 4 atomes de carbone éventuellement substitué par un radical phényle, ou un radical phényle, sur la 10-désacétyl-baccatine III pour obtenir un produit de formule générale : 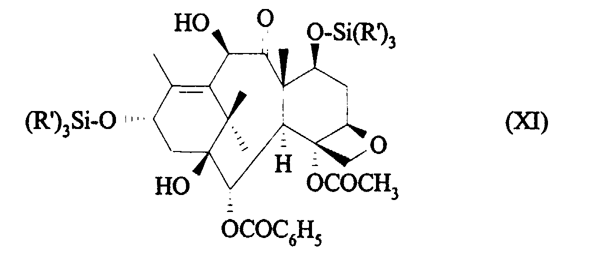 dans laquelle R' est défini comme précédemment, puis action d'un produit de formule générale :

R'₄-X₁ (XII)

dans laquelle R'₄ est tel que R'₄-O est identique à R₄ défini comme précédemment et X₁ représente un reste d'ester réactif ou un atome d'halogène pour obtenir un produit de formule générale : 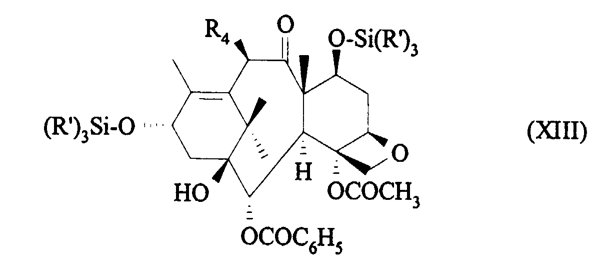 dans laquelle R' et R₄ sont définis comme précédemment, dont les groupements protecteurs silylés sont remplacés par des atomes d'hydrogène pour obtenir un produit de formule générale : 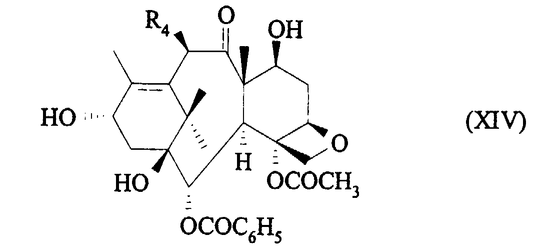 dans laquelle R₄ est défini comme précédemment, qui par traitement par un dérivé de l'acide trifluorométhanesulfonique tel que l'anhydride ou le N-phényl trifluoro-méthanesulfonimide dans un solvant organique inerte (hydrocarbures aliphatiques éventuellement halogénés, hydrocarbures aromatiques) en présence d'une base organique telle qu'une amine tertiaire aliphatique (triéthylamine) ou la pyridine à une température comprise entre -50 et +20°C, pour obtenir un produit de formule générale (III) dans laquelle R₄ est défini comme précédemment et R₅ représente un radical trifluorométhanesulfonyloxy, qui, par traitement éventuel par un halogénure de métal alcalin (iodure de sodium, fluorure de potassium) ou un azoture de métal alcalin (azoture de sodium) ou un sel d'ammonium en opérant dans un solvant organique choisi parmi les éthers (tétrahydrofuranne, diisopropyléther, méthyl t.butyléther), les nitriles (acétonitrile) ou les esters aliphatiques (acétate d'éthyle) seul ou en mélange à une température comprise entre 20°C et la température d'ébullition du mélange réactionnel, conduit à un produit de formule générale (III) dans laquelle R₄ est défini comme précédemment et R₅ représente une liaison avec l'atome de carbone du radical méthyle en α de façon à former un cycle cyclopropane, c'est-à-dire un produit de formule générale (I) dans laquelle Z représente un atome d'hydrogène.

Les nouveaux produits de formule générale (I) obtenus par la mise en oeuvre des procédés selon l'invention peuvent être purifiés selon les méthodes connues telles que la cristallisation ou la chromatographie.

Les produits de formule générale (I) dans laquelle Z représente un radical de formule générale (II) présentent des propriétés biologiques remarquables.

In vitro, la mesure de l'activité biologique est effectuée sur la tubuline extraite de cerveau de porc par la méthode de M.L. Shelanski et coll., Proc. Natl. Acad. Sci. USA, 70, 765-768 (1973). L'étude de la dépolymérisation des microtubules en tubuline est effectuée selon la méthode de G. Chauvière et coll., C.R. Acad. Sci., 293, série II, 501-503 (1981). Dans cette étude les produits de formule générale (I) dans laquelle Z représente un radical de formule générale (II) se sont montrés au moins aussi actifs que le taxol et le Taxotère.

In vivo, les produits de formule générale (I) dans laquelle Z représente un radical de formule générale (II) se sont montrés actifs chez la souris greffée par le mélanome B16 à des doses comprises entre 1 et 10 mg/kg par voie intrapéritonéale, ainsi que sur d'autres tumeurs liquides ou solides.

Les nouveaux produits ont des propriétés anti-tumorales et plus particulièrement une activité sur les tumeurs qui sont résistantes au Taxol® ou au Taxotère®. De telles tumeurs comprennent les tumeurs du colon qui ont une expression élevée du gène mdr 1 (gène de la multi-drug resistance). La multi-drug resistance est un terme habituel se rapportant à la résistance d'une tumeur à différents produits de structures et de mécanismes d'action différents. Les taxoïdes sont généralement connus pour être fortement reconnus par des tumeurs expérimentales telles que P388/DOX, une lignée cellulaire sélectionnée pour sa résistance à la doxorubicine (DOX) et qui surexprime mdr 1.

Les exemples suivants illustrent la présente invention.

### EXEMPLE 1

A une suspension de 0,504 g d'acétoxy-4α benzoyloxy-2α époxy-5β,20 dihydroxy-1β,13α méthoxy-10β oxo-9 trifluorométhanesulfonyloxy-7β taxène-11, 0,38 g d'acide tert-butoxycarbonyl-3 (méthoxy-4 phényl)-2 phényl-4 oxazolidine-1,3 carboxylique-5 (2R,4S,5R) et 0,1 g de tamis moléculaire 4Å activé en poudre dans 3,2 cm3 de toluène anhydre, on ajoute successivement, à une température voisine de 20°C, 0,24 g de dicyclohexylcarbodiimide et 30 mg de N,N'-diméthylamino-4 pyridine. Après une heure à une température voisine de 20°C, le mélange réactionnel est purifié (dépôt direct sur la colonne) par chromatographie à pression atmosphérique sur 50 g de silice (0,063-0,2 mm) contenus dans une colonne de 2,5 cm de diamètre (gradient d'élution : acétate d'éthyle-dichlorométhane de 0-100 à 10-90 en volumes) en recueillant des fractions de 10 cm3. Les fractions ne contenant que le produit cherché sont réunies et concentrées à sec sous pression réduite (0,27 kPa) à 40°C pendant 2 heures. On obtient ainsi 721,3 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α époxy-5β,20 hydroxy-1β méthoxy-10β oxo-9 trifluorométhanesulfonyloxy-7β taxène-11 yle-13α sous forme d'un solide jaune pâle dont les caractéristiques sont les suivantes :
- spectre de R.M.N. ¹H (400 MHz ; CDCl₃ ; δ en ppm ; constantes de couplage J en Hz) : 1,08 (s, 9H : C(CH₃)₃); 1,17 (s, 3H : CH₃); 1,21 (s, 3H : CH₃); 1,56 (s, 3H : CH₃); 1,60 (s, 1H : OH en 1); 1,71 (s, 3H : CH₃); 1,91 (s, 3H : COCH₃); 2,08 et 2,24 (2 dd, J = 16 et 9, 1H chacun : CH₂ en 14); de 2,15 à 2,30 et 2,78 (2 mts, 1H chacun : CH₂ en 6); 3,42 (s, 3H : OCH₃); 3,83 (s, 3H : ArOCH₃); 3,84 (d, J = 7, 1H : H en 3) ; 4,12 et 4,28 (2 d, J = 8,5, 1H chacun : CH₂ en 20) ; 4,58 (d, J = 5, 1H: H en 2') ; 4,85 (d large, J = 10, 1H : H en 5) ; 5,01 (s, 1H : H en 10) ; 5,40 (dd, J = 11 et 8, 1H : H en 7); 5,47 (mt, 1H : H en 3'); 5,65 (d, J = 7, 1H : H en 2); 6,12 (t large, J = 9, 1H : H en 13); 6,42 (mt, 1H : H en 5') ; 6,94 (d, J = 8,5, 2H : H aromatiques en ortho du OCH₃); de 7,20 à 7,45 (mt, 5H : H aromatiques en 3'); 7,42 (d, J = 8,5, 2H : H aromatiques en méta du OCH₃); 7,50 (t, J = 7,5, 2H : OCOC₆H₅ H en méta) ; 7,64 (t, J = 7,5, 1H : OCOC₆H₅ H en para) ; 8,02 (d, J = 7,5, 2H : OCOC₆H₅ H en ortho).

Une solution de 721 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α époxy-5β,20 hydroxy-1β méthoxy-10β oxo-9 trifluorométhanesulfonyloxy-7β taxène-11 yle-13α dans 13,5 cm3 d'une solution 0,1N d'éthanol chlorhydrique est maintenue sous agitation pendant 1 heure sous atmosphère d'argon à une température voisine de 0°C, puis pendant 4 heures à une température voisine de 20°C, et finalement pendant 16 heures à une température voisine de 0°C. Le mélange réactionnel est dilué avec 25 cm3 de dichlorométhane, lavé avec deux fois 5 cm3 d'eau distillée. La phase organique est séchée sur sulfate de magnésium, filtrée sur verre fritté puis concentrée à sec sous pression réduite (0,27 kPa) à une température voisine de 40°C. On obtient ainsi 704 mg d'une meringue jaune pâle que l'on purifie par chromatographie à pression atmosphérique sur 70 g de silice (0,063-0,2 mm) contenus dans une colonne de 2,5 cm de diamètre en éluant (gradient d'élution : acétate d'éthyle-dichlorométhane de 0-100 à 15-85 en volumes) en recueillant des fractions de 10 cm3. Les fractions ne contenant que le produit cherché sont réunies et concentrées à sec sous pression réduite (0,27 kPa) à 40°C pendant 2 heures. On obtient ainsi 539,5 mg de tert-butoxycarbonylamino-3 hydroxy-2 phényl-3 propionate-(2R,3S) d'acétoxy-4α benzoyloxy-2α époxy-5β,20 hydroxy-1β méthoxy-10β oxo-9 trifluorométhanesulfonyloxy-7β taxène-11 yle-13α sous forme d'une meringue ivoire dont les caractéristiques sont les suivantes :
- spectre de R.M.N. ¹H (400 MHz ; CDCl₃ ; δ en ppm ; constantes de couplage J en Hz) : 1,22 (s, 3H : CH₃); 1,25 (s, 3H : CH₃); 1,37 (s, 9H : C(CH₃)₃); 1,70 (s, 1H : OH en 1) ; 1,88 (s, 3H : CH₃); 1,95 (s, 3H : CH₃); 2,26 et 2,82 (2 mts, 1H chacun : CH₂ en 6); 2,32 (d, J = 9, 2H : CH₂ en 14); 2,41 (s, 3H : COCH₃); 3,36 (mf, 1H : OH en 2') ; 3,47 (s, 3H : OCH₃); 3,95 (d, J = 7, 1H : H en 3) ; 4,19 et 4,35 (2 d, J = 8,5, 1H chacun : CH₂ en 20); 4,63 (mt, 1H : H en 2'); 4,93 (d large, J = 10, 1H : H en 5) ; 5,11 (s, 1H : H en 10); 5,27 (d large, J = 10, 1H : H en 3') ; 5,40 (d, J = 10, 1H : CONH) ; 5,45 (dd, J = 10,5 et 8, 1H : H en 7); 5,70 (d, J = 7, 1H : H en 2); 6,26 (t large, J = 9, 1H : H en 13); de 7,25 à 7,45 (mt, 5H : H aromatiques en 3'); 7,51 (t, J = 7,5, 2H : OCOC₆H₅ H en méta) ; 7,64 (t, J = 7,5, 1H : OCOC₆H₅ H en para) ; 8,10 (d, J = 7,5, 2H : OCOC₆H₅ H en ortho).

A une solution de 265 mg de tert-butoxycarbonylamino-3 hydroxy-2 phényl-3 propionate-(2R,3S) d'acétoxy-4α benzoyloxy-2α époxy-5β,20 hydroxy-1β méthoxy-10β oxo-9 trifluorométhanesulfonyloxy-7β taxène-11 yle-13α dans 3 cm3 d'acétonitrile et 0,3 cm3 de tétrahydrofurane, on ajoute, à une température voisine de 20°C, 200 mg de tamis moléculaire 4Å en poudre, puis 300 mg de chlorure de sodium. Après 5 minutes à une température voisine de 20°C, la suspension obtenue, maintenue sous atmosphère d'argon, est porté au reflux pendant 3 heures. Après refroidissement jusqu'à une température voisine de 20°C, le mélange réactionnel est filtré sur verre fritté garni de célite. Après rinçage du verre fritté avec 30 cm3 d'un mélange acétate d'éthyle-dichlorométhane (50-50 en volumes), concentration à sec du filtrat sous pression réduite (0,27 kPa) à une température voisine de 40°C, on obtient 280 mg d'une meringue ivoire que l'on purifie par chromatographie préparative sur couche mince : 9 plaques préparatives Merck, Kieselgel 60F254, épaisseur 0,25mm, dépôt en solution dans le dichlorométhane, en éluant par un mélange méthanol-dichlorométhane (5-95 en volumes). Après élution de la zone correspondant au produit principal par un mélange méthanol-dichlorométhane (15-85 en volumes), filtration sur verre fritté, puis évaporation des solvants sous pression réduite (0,27 kPa) à une température voisine de 40°C, on obtient 142,6 mg de tert-butoxycarbonylamino-3 hydroxy-2 phényl-3 propionate-(2R,3S) d'acétoxy-4α benzoyloxy-2α époxy-5β,20 hydroxy-1β méthoxy-10β méthylène-7β,8β oxo-9 nor-19 taxène-11 yle-13α sous forme d'une meringue blanche dont les craractéristiques sont les suivantes :
- spectre de R.M.N. ¹H (400 MHz ; CDCl₃ ; température de 333°K ; δ en ppm ; constantes de couplage J en Hz) : 1,26 (s, 3H : CH₃); 1,29 (s, 3H : CH₃); 1,31 (s, 9H : C(CH₃)₃); 1,34 (mt, 1H : H en7) ; 1,65 et 2,33 (2 mts, 1H chacun : CH₂ en 19); 1,85 (s, 1H : OH en 1); 1,88 (s, 3H : CH₃); 2,12 et de 2,30 à 2,45 (respectivement d large et mt, J = 16, 1 H chacun : CH₂ en 6); 2,24 et de 2,30 à 2,45 (respectivement dd et mt, J = 16 et 9, 1H chacun : CH₂ en 14) ; 2,39 (s, 3H : COCH₃); 3,33 (mf, 1H : OH en 2') ; 3,47 (s, 3H : OCH₃); 4,06 et 4,32 (2 d, J = 8,5, 1H chacun : CH₂ en 20) ; 4,12 (d, J = 7, 1H : H en 3) ; 4,62 (mt, 1H : H en 2') ; 4,74 (d, J = 4, 1H : H en 5) ; 4,75 (s, 1H : H en 10) ; 5,28 (d large, J = 10, 1H : H en 3'); 5,37 (d, J = 10, 1H : CONH) ; 5,68 (d, J = 7, 1H : H en 2) ; 6,32 (t large, J = 9, 1H : H en 13); de 7,25 à 7,45 (mt, 5H : H aromatiques en 3'); 7,52 (t, J = 7,5, 2H : OCOC₆H₅ H en méta) ; 7,62 (t, J = 7,5, 1H : OCOC₆H₅ H en para) ; 8,16 (d, J = 7,5, 2H : OCOC₆H₅ H en ortho).

L'acétoxy-4α benzoyloxy-2α époxy-5β,20 dihydroxy-1β,13α méthoxy-10β oxo-9 trifluorométhanesulfonyloxy-7β taxène-11 peut être préparé de la manière suivante :

A une suspension de 0,5 g d'acétoxy-4α benzoyloxy-2α époxy-5β,20 trihydroxy-1β,7β,13α méthoxy-10β oxo-9 taxène-11 dans 10 cm3 de dichlorométhane anhydre et 0,3 cm3 de pyridine anhydre, refroidie à une température voisine de 0°C, maintenue sous atmosphère d'argon, on ajoute goutte à goutte 0,31 cm3 d'anhydride trifluorométhanesulfonique. Le mélange réactionnel est agité à une température voisine de 20°C pendant une heure, puis dilué avec 20 cm3 de dichlorométhane et 5 cm3 d'eau distillée. Après décantation, la phase aqueuse est réextraite avec deux fois 5 cm3 de dichlorométhane. Les phases organiques sont rassemblées, séchées sur sulfate de magnésium, filtrées sur verre fritté et concentrées à sec sous pression réduite (0,27 kPa) à une température voisine de 40°C. On obtient ainsi 0,79 g d'un solide orange que l'on purifie par chromatographie à pression atmosphérique sur 50 g de silice (0,063-0,2 mm) contenus dans une colonne de 2,5 cm de diamètre (gradient d'élution : acétate d'éthyle-dichlorométhane de 0-100 à 25-75 en volumes) en recueillant des fractions de 15 cm3. Les fractions ne contenant que le produit cherché sont réunies et concentrées à sec sous pression réduite (0,27 kPa) à 40°C pendant 2 heures. On obtient ainsi 504 mg d'acétoxy-4α benzoyloxy-2α époxy-5β,20 dihydroxy-1β,13α méthoxy-10β oxo-9 trifluorométhanesulfonyloxy-7β taxène-11 sous forme d'un solide blanc dont les caractéristiques sont les suivantes :
- spectre de R.M.N. ¹H (400 MHz; CDCl₃ ; δ en ppm ; constantes de couplage J en Hz) : 1,10 (s, 3H : CH₃); 1,19 (s, 3H : CH₃); 1,86 (s, 3H : CH₃); 2,09 (d, J = 5, 1H: OH en 13); 2,15 (s, 3H : CH₃); 2,25 et 2,85 (2 mts, 1H chacun : CH₂ en 6); 2,32 (d, J = 9, 2H : CH₂ en 14); 2,33 (s, 3H : COCH₃); 3,48 (s, 3H : OCH₃); 4,03 (d, J = 7, 1H : H en 3) ; 4,18 et 4,35 (2 d, J = 8,5, 1H chacun : CH₂ en 20) ; 4,92 (mt, 1H : H en 13); 4,96 (d large, J = 10, 1H : H en 5) ; 5,16 (s, 1H : H en 10) ; 5,53 (dd, J = 11 et 7, 1H : H en 7); 5,66 (d, J = 7, 1H : H en 2); 7,49 (t, J = 7,5, 2H : OCOC₆H₅ H en méta) ; 7,64 (t, J = 7,5, 1H : OCOC₆H₅ H en para) ; 8,10 (d, J = 7,5, 2H : OCOC₆H₅ H en ortho).

L'acétoxy-4α benzoyloxy-2α époxy-5β,20 trihydroxy-1β,7β,13α méthoxy-10β oxo-9 taxène-11 peut être préparé de la manière suivante :

A une solution de 3,62 g d'acétoxy-4α benzoyloxy-2α époxy-5β,20 hydroxy-1β méthoxy-10β oxo-9 bis(triéthylsilyloxy)-7β,13α taxène-11 dans 30 cm3 de dichlorométhane, maintenue sous atmosphère d'argon, à une température voisine de 0°C, on ajoute lentement 50 cm3 de complexe fluorure d'hydrogène-triéthylamine (3HF.Et₃N). Après 48 heures à une température voisine de 20°C, le mélange réactionnel est versé sur une suspension de 100 cm3 d'une solution aqueuse sursaturée en hydrogénocarbonate de sodium maintenue à une température voisine de 0°C. Après décantation, la phase aqueuse est extraite avec trois fois 80 cm3 de dichlorométhane, puis deux fois 80 cm3 d'acétate d'éthyle. Les phases organiques sont rassemblées, séchées sur sulfate de magnésium, filtrées sur sulfate de magnésium et concentrées à sec sous pression réduite (0,27 kPa) à une température voisine de 40°C. On obtient ainsi 3,45 g d'une meringue jaune que l'on purifie par chromatographie à pression atmosphérique sur 150 g de silice (0,063-0,2 mm) contenus dans une colonne de 3,5 cm de diamètre en éluant avec un mélange méthanol-dichlorométhane (5-95 en volumes) en recueillant des fractions de 35 cm3. Les fractions ne contenant que le produit cherché sont réunies et concentrées à sec sous pression réduite (0,27 kPa) à 40°C pendant 2 heures. On obtient ainsi 1,97 g d'acétoxy-4α benzoyloxy-2α époxy-5β,20 trihydroxy-1β,7β,13α méthoxy-10β oxo-9 taxène-11 sous forme d'un solide blanc dont les caractéristiques sont les suivantes :
- spectre de R.M.N. ¹H (400 MHz ; CDCl₃ ; δ en ppm ; constantes de couplage J en Hz): 1,10 (s, 3H : CH₃); 1,19 (s, 3H : CH₃); 1,48 (d, J = 8,5, 1H : OH en 7); 1,70 (s, 3H : CH₃) ; 1,81 et 2,61 (2 mts, 1H chacun : CH₂ en 6); 2,09 (d, J = 5, 1H : OH en 13); 2,11 (s, 3H : CH₃); 2,30 (s, 3H : COCH₃); 2,32 (d, J = 9, 2H : CH₂ en 14); 3,48 (s, 3H : OCH₃); 3,97 (d, J = 7, 1H : H en 3) ; 4,18 et 4,33 (2 d, J = 8,5, 1H chacun : CH₂ en 20) ; 4,31 (mt, 1H : H en 7); 4,93 (mt, 1H : H en 13); 4,99 (s, 1H : H en 10); 5.01 (d large, J = 10, 1H : H en 5) ; 5,66 (d, J = 7, 1H : H en 2); 7,49 (t, J = 7,5, 2H : OCOC₆H₅ H en méta) ; 7,63 (t, J = 7,5, 1H : OCOC₆H₅ H en para) ; 8,12 (d, J = 7,5, 2H : OCOC₆H₅ H en ortho).

L'acétoxy-4α benzoyloxy-2α époxy-5β,20 hydroxy-1β méthoxy-10β oxo-9 bis(triéthylsilyloxy)-7β,13α taxène-11 peut être préparé de la manière suivante :

A une solution de 5 g d'acétoxy-4α benzoyloxy-2α époxy-5β,20 dihydroxy-1β,10β oxo-9 bis(triéthylsilyloxy)-7β,13α taxène-11 dans 25 cm3 d'iodométhane, maintenue sous atmosphère d'argon, à une température voisine de 0°C, on ajoute par portions 375 mg d'hydrure de sodium à 50 % en poids dans l'huile de vaseline. La solution est maintenue sous agitation 45 minutes à une température voisine de 0°C, puis 5 heures 30 minutes à une température voisine de 20°C. Le mélange réactionnel est de nouveau refroidi à une température voisine de 0°C, et on ajoute par portions 125 mg d'hydrure de sodium à 50 % en poids dans l'huile de vaseline. Après 1 heure à 20°C, puis 18 heures à 5°C, le mélange réactionnel est dilué avec 50 cm3 de dichlorométhane, versé sur 50 cm3 d'une solution aqueuse saturée en chlorure d'ammonium et décanté. La phase aqueuse est réextraite par deux fois 30 cm3 de dichlorométhane, puis les phases organiques sont rassemblées, lavées avec 10 cm3 d'eau distillée, séchées sur sulfate de magnésium, filtrées sur verre fritté, et concentrées à sec sous pression réduite (0,27 kPa) à une température voisine de 40°C. On obtient ainsi 5,15 g d'une meringue jaune que l'on purifie par chromatographie à pression atmosphérique sur 300 g de silice (0,063-0,2 mm) contenus dans une colonne de 5 cm de diamètre (gradient d'élution : acétate d'éthyle-dichlorométhane de 0-100 à 10-90 en volumes) en recueillant des fractions de 30 cm3. Les fractions ne contenant que le produit cherché sont réunies et concentrées à sec sous pression réduite (0,27 kPa) à 40°C pendant 2 heures. On obtient ainsi 3,62 g d'acétoxy-4α benzoyloxy-2α époxy-5β,20 hydroxy-1β méthoxy-10β oxo-9 bis(triéthylsilyloxy)-7β,13α taxène-11 sous forme d'une meringue jaune pâle dont les caractéristiques sont les suivantes :
- spectre de R.M.N. ¹H (600 MHz; CDCl₃ ; δ en ppm ; constantes de couplage J en Hz) : 0,58 et 0,69 (2 mts, 6H chacun : CH₂ éthyle) ; 0,97 et 1,04 (2 t, J = 7,5, 9H chacun : CH₃ éthyle) ; 1,15 (s, 3H : CH₃); 1,18 (s, 3H : CH₃); 1,58 (s, 1H : 0H en 1) ; 1,68 (s, 3H : CH₃) ; 1,89 et 2,48 (2 mts, 1H chacun : CH₂ en 6); 2,04 (s, 3H : CH₃); 2,15 et 2,23 (2 dd, J = 16 et 9, 1H chacun : CH₂ en 14); 2,29 (s, 3H : COCH₃); 3,40 (s, 3H : OCH₃); 3,83 (d, J = 7, 1H : H en 3) ; 4,15 et 4,30 (2 d, J = 8,5, 1H chacun : CH₂ en 20) ; 4,43 (dd, J = 11 et 7, 1H : H en 7) ; 4,91 (s, 1H : H en 10) ; 4,96 (d large, J = 10, 1H: H en 5) ; 5,01 (t large, J = 9, 1H : H en 13) ; 5,62 (d, J = 7, 1H : H en 2) ; 7,46 (t, J = 7,5, 2H : OCOC₆H₅ H en méta); 7,60 (t, J = 7,5, 1H: OCOC₆H ₅H en para) ; 8,09 (d, J = 7,5, 2H : OCOC₆H₅ H en ortho).

L'acétoxy-4α benzoyloxy-2α époxy-5β,20 dihydroxy-1β,10β oxo-9 bis(triéthylsilyloxy)-7β,13α taxène-11 peut être préparé de la manière suivante :

A une solution de 14 g d'acétoxy-4α benzoyloxy-2α époxy-5β,20 tétrahydroxy-1β,7β,10β,13α oxo-9 taxène-11 dans 50 cm3 de pyridine anhydre, maintenue sous atmosphère d'argon, à une température voisine de 20°C, on ajoute 10,8 cm3 de chlorure de triéthylsilyle. Après 17 heures à une température voisine de 20°C, le mélange réactionnel est porté à une température voisine de 115°C, puis on ajoute 10,8 cm3 de chlorure de triéthylsilyle. Après 3 heures 15 minutes à une température voisine de 115°C, le mélange réactionnel est ramené jusqu'à une température voisine de 20°C, dilué avec 300 cm3 d'acétate d'éthyle et 100 cm3 d'eau distillée. Après décantation, la phase aqueuse est réextraite avec deux fois 50 cm3 d'acétate d'éthyle. Les phases organiques sont rassemblées, lavées avec 50 cm3 d'une solution aqueuse saturée en chlorure de sodium, séchées sur sulfate de magnésium, filtrées sur verre fritté puis concentrées à sec sous pression réduite (0,27 kPa) à une température voisine de 40°C. On obtient ainsi 63,1 g d'une huile brune que l'on purifie par chromatographie à pression atmosphérique sur 800 g de silice (0,063-0,2 mm) contenus dans une colonne de 7 cm de diamètre (gradient d'élution : acétate d'éthyle-dichlorométhane de 0-100 à 5-95 en volumes) en recueillant des fractions de 60 cm3. Les fractions ne contenant que le produit cherché sont réunies et concentrées à sec sous pression réduite (0,27 kPa) à 40°C pendant 2 heures. On obtient ainsi 9,77 g d'acétoxy-4α benzoyloxy-2α époxy-5β,20 dihydroxy-1β,10β oxo-9 bis(triéthylsilyl-oxy)-7β,13α taxène-11 sous forme d'une meringue crème dont les caractéristiques sont les suivantes :
- spectre de R.M.N. ¹H (400 MHz ; CDCl₃ ; δ en ppm ; constantes de couplage J en Hz): 0,55 et 0,68 (2 mts, 6H chacun : CH₂ éthyle) ; 0,94 et 1,03 (2 t, J = 7,5, 9H chacun : CH₃ éthyle) ; 1,08 (s, 3H : CH₃); 1,17 (s, 3H : CH₃); 1,58 (s, 1H: 0H en 1); 1,73 (s, 3H : CH₃) ; 1,91 et 2,57 (2 mts, 1H chacun : CH₂ en 6); 2,04 (s, 3H : CH₃); 2,12 et 2,23 (2 dd, J = 16 et 9, 1H chacun : CH₂ en 14) ; 2,30 (s, 3H : COCH₃); 3,88 (d, J = 7, 1H: H en 3) ; 4,16 et 4,32 (2 d, J = 8,5, 1H chacun : CH₂ en 20) ; 4,27 (d, J = 1, 1H: OH en 10) ; 4,40 (dd, J = 11 et 7, 1H : H en 7); 4,95 (d large, J = 10, 1H : H en 5); 4,95 (mt, 1H : H en 13); 5,16 (d, J = 1, 1H: H en 10) ; 5,60 (d, J = 7, 1H: H en 2) ; 7,46 (t, J = 7,5, 2H : OCOC₆H₅ H en méta) ; 7,60 (t, J = 7,5, 1H: OCOC₆H₅ H en para) ; 8,09 (d, J = 7,5, 2H : OCOC₆H₅ H ortho).

### EXEMPLE 2

A une solution de 250 mg de tert-butoxycarbonylamino-3 hydroxy-2 phényl-3 propionate-(2R,3S) d'acétoxy-4α benzoyloxy-2α époxy-5β,20 hydroxy-1β éthoxy-10β oxo-9 trifluorométhanesulfoyloxy-7β taxène-11 yle-13α dans 2,5 cm3 d'acétonitrile et 0,25 cm3 de tétrahydrofurane, on ajoute, à une température voisine de 20°C, 100 mg de tamis moléculaire 4Å en poudre, puis 200 mg de chlorure de sodium. Après 10 minutes à une température voisine de 20°C, la suspension obtenue, maintenue sous atmosphère d'argon, est porté au reflux pendant 2 heures. Après refroidissement jusqu'à une température voisine de 20°C, le mélange réactionnel est dilué avec 50 cm3 d'acétate d'éthyle et filtré sur verre fritté garni de célite. Après rinçage du verre fritté avec 10 cm3 d'acétate d'éthyle, lavage du filtrat avec deux fois 10 cm3 d'une solution aqueuse saturée en hydrogénocarbonate de sodium, deux fois 10 cm3 d'eau distillée, deux fois 10 cm3 d'une solution aqueuse saturée en chlorure de sodium, séchage sur sulfate de magnésium, filtration sur verre fritté et concentration à sec du filtrat sous pression réduite (2,7 kPa) à une température voisine de 40°C, on obtient 209 mg d'une meringue jaune que l'on purifie par chromatographie préparative sur couche mince : 9 plaques préparatives Merck, Kieselgel 60F254, épaisseur 0,5 mm, dépôt en solution dans le dichlorométhane, en éluant par un mélange méthanol-dichlorométhane (5-95 en volumes). Après élution de la zone correspondant au produit principal par un mélange méthanol-dichlorométhane (15-85 en volumes), filtration sur verre fritté, puis évaporation des solvants sous pression réduite (2,7 kPa) à une température voisine de 40°C, on obtient 66,5 mg de tert-butoxycarbonylamino-3 hydroxy-2 phényl-3 propionate-(2R,3S) d'acétoxy-4α benzoyloxy-2α époxy-5β,20 hydroxy-1β éthoxy-10β méthylène-7β,8β oxo-9 nor-19 taxène-11 yle-13α sous forme d'une meringue ivoire dont les caractéristiques sont les suivantes :
- spectre de R.M.N. ¹H (300 MHz ; CDCl₃ ; déplacements chimiques δ en ppm ; constantes de couplage J en Hz) ; 1,25 (s, 3H : CH₃); 1,33 (t, J = 7, 3H : CH₃ de l'éthyle) ; 1,34 (s, 9H : C(CH₃)₃); 1,35 (s, 3H : CH₃); 1,37 (mt, 1H: H en 7); de 1,55 à 1,70 et de 2,20 à 2,40 (2 mts, 1H chacun : CH₂ en 19); 1,80 (s, 1H : OH en 1); 1,85 (s, 3H : CH₃); 2,10 et 2,40 (respectivement d large et dt, J = 15 et J = 15 et 4, 1H chacun : CH₂ en 6); 2,20 et de 2,20 à 2,40 (respectivement dd et mt, J = 16 et 9, 1H chacun : CH₂ en 14); 2,38 (s, 3H : COCH₃); 3,30 (mt, 1H: OH en 2'); 3,60 (AB limite, 2H : OCH₂ de l'éthyle) ; 4,05 et 4,30 (2 d, J = 8,5, 1H chacun : CH₂ en 20) ; 4,13 (d, J = 7,5, 1H : H en 3) ; 4,62 (mt, 1H : H en 2') ; 4,74 (d large, J = 4, 1H : H en 5) ; 4,83 (s, 1H : H en 10) ; 5,27 (d large, J = 10, 1H : H en 3') ; 5,35 (d, J = 10, 1H : CONH) ; 5,67 (d, J = 7,5, 1H : H en 2) ; 6,30 (t large, J = 9, 1H : H en 13) ; de 7,25 à 7,45 (mt, 5H : H aromatiques en 3'); 7,52 (t, J = 7,5, 2H : OCOC₆H₅ H en méta) ; 7,63 (t, J = 7,5, 1H : OCOC₆H₅ H en para) ; 8,15 (d, J = 7,5, 2H : OCOC₆H₅ H en ortho).

Le tert-butoxycarbonylamino-3 hydroxy-2 phényl-3 propionate-(2R,3S) d'acétoxy-4α benzoyloxy-2α époxy-5β,20 hydroxy-1β éthoxy-10β oxo-9 trifluorométhanesulfonyloxy-7β taxène-11 yle-13α peut être préparé de la manière suivante :

Une solution de 423 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α époxy-5β,20 hydroxy-1β éthoxy-10β oxo-9 trifluorométhanesulfonyloxy-7β taxène-11 yle-13α dans 7 cm3 d'une solution 0,1N d'éthanol chlorhydrique est maintenue pendant 15 heures sous atmosphère d'argon à une température voisine de 0°C. Le mélange réactionnel est dilué avec 35 cm3 de dichlorométhane, lavé avec deux fois 7 cm3 d'eau distillée puis 7 cm3 d'une solution aqueuse saturée en chlorure de sodium. La phase organique est séchée sur sulfate de magnésium, filtrée sur verre fritté puis concentrée à sec sous pression réduite (2,7 kPa) à une température voisine de 40°C. On obtient ainsi 358 mg d'un solide brun que l'on purifie par chromatographie à pression atmosphérique sur 30 g de silice (0,063-0,2 mm) contenus dans une colonne de 2,5 cm de diamètre en éluant (gradient d'élution : méthanol-dichlorométhane de 1-99 à 5-95 en volumes) en recueillant des fractions de 10 cm3. Les fractions ne contenant que le produit cherché sont réunies et concentrées à sec sous pression réduite (2,7 kPa) à 40°C pendant 2 heures. On obtient ainsi 263,1 mg de tert-butoxycarbonylamino-3 hydroxy-2 phényl-3 propionate-(2R,3S) d'acétoxy-4α benzoyloxy-2α époxy-5β,20 hydroxy-1β éthoxy-10β oxo-9 trifluorométhanesulfonyloxy-7β taxène-11 yle-13α sous forme d'une meringue crème dont les caractéristiques sont les suivantes :
- spectre de R.M.N. ¹H (300 MHz ; CDCl₃ ; déplacements chimiques δ en ppm ; constantes de couplage J en Hz) : 1,25 (s, 3H : CH₃); 1,26 (s, 3H : CH₃), 1,27 (t, J = 7, 3H : CH₃ de l'éthyle) ; 1,37 (s, 9H : C(CH₃)₃); 1,66 (s, 1H : OH en 1) ; 1,85 (s, 3H : CH₃); 1,95 (s, 3H : CH₃); 2,25 et 2,80 (2 mts, 1H chacun : CH₂ en 6); 2,30 (d, J = 9, 2H : CH₂ en 14); 2,40 (s, 3H : COCH₃); 3,35 (d, J = 4, 1H : OH en 2'); 3,55 et 3,65 (2 mts, 1H chacun : OCH₂ de l'éthyle) ; 3,95 (d, J = 7,5 Hz, 1H : H en 3) ; 4,17 et 4,35 (2 d, J = 8,5, 1H chacun : CH₂ en 20); 4,62 (mt, 1H:H en 2'); 4,93 (d large, J = 10, 1H : H en 5) ; 5,17 (s, 1H : H en 10); 5,27 (d large, J = 10, 1H: H en 3'); 5,37 (d, J = 10, 1H: CONH) ; 5,45 (dd, J = 11 et 6,5, 1H : H en 7); 5,73 (d, J = 7,5, 1H : H en 2); 6,25 (t large, J = 9, 1H: H en 13); de 7,25 à 7,45 (mt, 5H : H aromatiques en 3'); 7,52 (t, J = 7,5, 2H : OCOC₆H₅ H en méta) ; 7,63 (t, J = 7,5, 1H : OCOC₆H₅ H en para) ; 8,10 (d, J = 7,5, 2H : OCOC₆H₅ H en ortho).

Le tert-butoxycarbonyl-3 (méthoxy-4 phényl)-2 phényl-4 oxazolidine-1,3 carboxylate-5 (2R,4S,5R) d'acétoxy-4α benzoyloxy-2α époxy-5β,20 hydroxy-1β éthoxy-10β oxo-9 trifluorométhanesulfonyloxy-7β taxène-11 yle-13α peut être préparé de la manière suivante :

A une suspension de 238 mg d'acétoxy-4α benzoyloxy-2α époxy-5β,20 dihydroxy-1β,13α éthoxy-10β oxo-9 trifluorométhanesulfonyloxy-7β taxène-11, 182 mg d'acide tert-butoxycarbonyl-3 (méthoxy-4 phényl)-2 phényl-4 oxazolidine-1,3 carboxylique-5-(2R,4S,5R) dans 2 cm3 de toluène anhydre et 0,2 cm3 de dichlorométhane, on ajoute successivement, à une température voisine de 20°C, 116 mg de dicyclohexylcarbodiimide et 13 mg de N,N'-diméthylamino-4 pyridine. Après 45 minutes à une température voisine de 20°C, le mélange réactionnel est purifié (dépôt direct sur la colonne) par chromatographie à pression atmosphérique sur 50 g de silice (0,063-0,2 mm) contenus dans une colonne de 2,5 cm de diamètre en éluant avec un mélange méthanol-dichlorométhane (1-99 en volumes) en recueillant des fractions de 10 cm3. Les fractions ne contenant que le produit cherché sont réunies et concentrées à sec sous pression réduite (0,27 kPa) à 40°C pendant 2 heures. On obtient ainsi 443,6 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β éthoxy-10β oxo-9 trifluorométhanesulfonyloxy-7β taxène-11 yle-13α sous forme d'une meringue jaune pâle.

L'acétoxy-4α benzoyloxy-2α époxy-5β,20 dihydroxy-1β,13α éthoxy-10β oxo-9 trifluorométhanesulfonyloxy-7β taxène-11 peut être préparé de la manière suivante :

A une suspension de 199 mg d'acétoxy-4α benzoyloxy-2α époxy-5β,20 trihydroxy-1β,7β,13α éthoxy-10β oxo-9 taxène-11 dans 2 cm3 de dichlorométhane anhydre et 0,14 cm3 de pyridine anhydre, refroidie à une température voisine de 0°C, maintenue sous atmosphère d'argon, on ajoute goutte à goutte 0,145 cm3 d'anhydride trifluorométhanesulfonique. Le mélange réactionnel est agité à une température voisine de 0°C pendant une heure, puis on ajoute goutte à goutte 0,07 cm3 d'anhydride trifluorométhanesulfonique. Le mélange réactionnel est agité à une température voisine de 0°C pendant 1,5 heure, dilué avec 1 cm3 d'un mélange méthanol-dichlorométhane (5-95 en volumes), concentré à sec sous pression réduite (2,7 kPa) à une température voisine de 30°C. Le résidu brut obtenu est purifié par chromatographie à pression atmosphérique sur 30 g de silice (0,063-0,2 mm) contenus dans une colonne de 2,5 cm de diamètre en éluant avec un mélange méthanol-dichlorométhane (2-98 en volumes) en recueillant des fractions de 10 cm3. Les fractions ne contenant que le produit cherché sont réunies et concentrées à sec sous pression réduite (2,7 kPa) à 40°C pendant 2 heures. On obtient ainsi 238,2 mg d'acétoxy-4α benzoyloxy-2α époxy-5β,20 dihydroxy-1β,13α éthoxy-10β oxo-9 trifluorométhanesulfonyloxy-7β taxène-11 sous forme d'une meringue jaune.

L'acétoxy-4α benzoyloxy-2α époxy-5β,20 trihydroxy-1β,7β,13α éthoxy-10β oxo-9 taxène-11 (ou éthoxy-10β désacétoxy-10 baccatine III) peut être préparé de la manière suivante :

A une solution de 591 mg d'acétoxy-4α benzoyloxy-2α époxy-5β,20 hydroxy-1β éthoxy-10β oxo-9 bistriéthylsilyloxy-7β,13α taxène-11 dans 6 cm3 de dichlorométhane, maintenue sous atmosphère d'argon, à une température voisine de 20°C, on ajoute 9 cm3 de complexe fluorure d'hydrogène-triéthylamine (3HF.Et₃N). Après 21 heures à une température voisine de 20°C, le mélange réactionnel est dilué avec 40 cm3 de dichlorométhane et versé sur une suspension de 40 cm3 d'une solution aqueuse sursaturée en NaHCO₃, maintenue à une température voisine de 0°C. Après dilution avec 10 cm3 d'eau distillée et décantation, la phase aqueuse est réextraite avec deux fois 20 cm3 de diéthyléther. Les phases organiques sont rassemblées, lavées avec 20 cm3 d'eau distillée, 20 cm3 d'une solution aqueuse saturée en chlorure de sodium, séchées sur sulfate de magnésium, filtrées sur sulfate de magnésium et concentrées à sec sous pression réduite (2,7 kPa) à une température voisine de 40°C. On obtient ainsi 370 mg d'une meringue jaune pâle que l'on purifie par chromatographie à pression atmosphérique sur 35 g de silice (0,063-0,2 mm) contenus dans une colonne de 2,5 cm de diamètre en éluant avec un mélange méthanol-dichlorométhane (2-98 en volumes) en recueillant des fractions de 15 cm3. Les fractions ne contenant que le produit cherché sont réunies et concentrées à sec sous pression réduite (2,7 kPa) à 40°C pendant 2 heures. On obtient ainsi 236,2 mg d'acétoxy-4α benzoyloxy-2α époxy-5β,20 trihydroxy-1β,7β,13α éthoxy-10β oxo-9 taxène-11 sous forme d'un solide blanc dont les caractéristiques sont les suivantes :
- spectre de R.M.N. ¹H (400 MHz ; CDCl₃ ; déplacements chimiques δ en ppm ; constantes de couplage J en Hz) : 1,08 (s, 3H : CH₃); 1,19 (s, 3H : CH₃); 1,29 (t, J = 7,5, 3H : CH₃ éthyle) ; 1,38 (d, J = 9, 1H : OH en 7); 1,59 (s, 1H : OH en 1); 1,69 (s, 3H : CH₃); 1,82 et 2,62 (2 mts, 1H chacun : CH₂ en 6); 2,02 (d, J = 5, 1H : OH en 13); 2,08 (s, 3H : CH₃); 2,30 (s, 3H : COCH₃); 2,32 (d, J = 9, 2H : CH₂ en 14); 3,56 et 3,67 (2 mts, 1H chacun : OCH₂ éthyle) ; 3,98 (d, J = 7, 1H : H en 3); 4,18 et 4,33 (2 d, J = 8,5, 1H chacun : CH₂ en 20); 4,30 (mt, 1H : H en 7); 4,90 (mt, 1H: H en 13); 4,99 (dd, J = 10 et 1,5, 1H : H en 5) ; 5,05 (s, 1H : H en 10); 5,66 (d, J = 7, 1H : H en 2); 7,49 (t, J = 7,5, 2H : OCOC₆H₅ H en méta) ; 7,63 (t, J = 7,5, 1H: OCOC₆H₅ H en para) ; 8,12 (d, J = 7,5, 2H : OCOC₆H₅ H en ortho).

L'acétoxy-4α benzoyloxy-2α époxy-5β,20 hydroxy-1β éthoxy-10β oxo-9 bistriéthylsilyloxy-7β,13α taxène-11 (ou éthoxy-10β désacétoxy-10 bistriéthylsilyl-7,13 baccatine III) peut être préparé de la manière suivante :

A une solution de 1 g d'acétoxy-4α benzoyloxy-2α époxy-5β,20 dihydroxy-1β,10β oxo-9 bistriéthylsilyloxy-7β,13α taxène-11 dans 3 cm3 d'iodoéthane et 4 cm3 de diméthylformamide, maintenue sous atmosphère d'argon, à une température voisine de 20°C, on ajoute par portions 93 mg d'hydrure de sodium à 50 % en poids dans l'huile de vaseline. La solution est maintenue sous agitation pendant 17 heures à une température voisine de 20°C, puis on ajoute par portions 93 mg d'hydrure de sodium à 50 % en poids dans l'huile de vaseline. Après 50 minutes à une température voisine de 20°C le mélange réactionnel est dilué avec 100 cm3 d'acétate d'éthyle, 10 cm3 d'une solution aqueuse saturée en chlorure d'ammonium. La phase organique décantée est lavée avec six fois 10 cm3 d'eau distillée, puis 10 cm3 d'une solution aqueuse saturée en chlorure de sodium, séchée sur sulfate de magnésium, filtrée sur verre fritté, et concentrée à sec sous pression réduite (2,7 kPa) à une température voisine de 40°C. On obtient ainsi 1,2 g d'une meringue jaune que l'on purifie par chromatographie à pression atmosphérique sur 150 g de silice (0,063-0,2 mm) contenus dans une colonne de 3,5 cm de diamètre en éluant avec un mélange acétate d'éthyle-dichlorométhane (2-98 puis 5-95 en volumes) en recueillant des fractions de 15 cm3. Les fractions ne contenant que les produits cherchés sont réunies et concentrées à sec sous pression réduite (0,27 kPa) à 40°C pendant 2 heures. On obtient ainsi 379,2 mg d'acétoxy-4α benzoyloxy-2α époxy-5β,20 dihydroxy-1β,10β oxo-9 bistriéthylsilyloxy-7β,13α taxène-11 sous forme d'une meringue jaune pâle et 430 mg d'acétoxy-4α benzoyloxy-2α époxy-5β,20 hydroxy-1β éthoxy-10β oxo-9 bistriéthylsilyloxy-7β,13α taxène-11 sous forme d'une meringue blanche dont les caractéristiques sont les suivantes :
- spectre de R.M.N. ¹H (400 MHz ; CDCl₃ ; déplacements chimiques δ en ppm ; constantes de couplage J en Hz) : 0,57 et 0,70 (2 mts, 6H chacun : CH₂ de l'éthyle) ; 0,97 et 1,03 (2 t, J = 7,5, 9H chacun : CH₃ de l'éthyle) ; 1,13 (s, 3H : CH₃); 1,20 (s, 3H : CH₃); 1,29 (t, J = 7,5, 3H : CH₃ de l'éthoxy en 10); 1,58 (s, 1H : OH en 1); 1,66 (s, 3H : CH₃); 1,89 et 2,58 (2 mts, 1H chacun : CH₂ en 6); 2,03 (s, 3H : CH₃); 2,13 et 2,23 (2 dd, J =16 et 9, 1H chacun : CH₂ en 14); 2,30 (s, 3H: COCH₃); 3,53 (mt, 2H : CH₂ de l'éthoxy en 10); 3,84 (d, J = 7 Hz, 1H : H en 3) ; 4,15 et 4,30 (2 d, J = 8,5, 1H chacun : CH₂ en 20) ; 4,43 (dd, J = 11 et 6,5, 1H : H en 7) ; de 4,90 à 5,00 (mt, 2H : H en 13 et H en 5) ; 5,01 (s, 1H : H en 10); 5,61 (d, J = 7, 1H : H en 2) ; 7,48 (t, J = 7,5, 2H : OCOC₆H₅ H en méta) ; 7,61 (t, J = 7,5, 1H : OCOC₆H₅ H en para) ; 8,10 (d, J = 7,5, 2H : OCOC₆H₅ H en ortho).

### EXEMPLE 3

A une solution de 80 mg de tert-butoxycarbonylamino-3 hydroxy-2 phényl-3 propionate-(2R,3S) d'acétoxy-4α benzoyloxy-2α époxy-5β,20 hydroxy-1β (propyl-1) oxy-10β oxo-9 trifluorométhanesulfonyloxy-7β taxène-11 yle-13α dans 0,8 cm3 d'acétonitrile et 0,8 cm3 de tétrahydrofurane, on ajoute, à une température voisine de 20°C, 50 mg de tamis moléculaire 4Å en poudre, puis 80 mg de chlorure de sodium. Après 5 minutes à une température voisine de 20°C, la suspension obtenue, maintenue sous atmosphère d'argon, est porté au reflux pendant 2,5 heures. Après refroidissement jusqu'à une température voisine de 20°C, le mélange réactionnel est filtré sur célite. Après rinçage de la célite avec 5 cm3 d'acétate d'éthyle et concentration à sec du filtrat sous pression réduite (2,7 kPa) à une température voisine de 40°C, on obtient 81,2 mg d'une meringue jaune que l'on purifie par chromatographie préparative sur couche mince : 5 plaques préparatives Merck, Kieselgel 60F254, épaisseur 0,5 mm, dépôt en solution dans le dichlorométhane, en éluant par un mélange méthanol-dichlorométhane (5-95 en volumes). Après élution de la zone correspondant au produit principal par un mélange méthanol-dichlorométhane (15-85 en volumes), filtration sur verre fritté, puis évaporation des solvants sous pression réduite (2,7 kPa) à une température voisine de 40°C, on obtient 36,5 mg de tert-butoxycarbonylamino-3 hydroxy-2 phényl-3 propionate-(2R,3S) d'acétoxy-4α benzoyloxy-2α époxy-5β,20 hydroxy-1β (propyl-1)oxy-10β méthylène-7β,8β oxo-9 nor-19 taxène-11 yle-13α sous forme d'une meringue ivoire dont les caractéristiques sont les suivantes :
- spectre de R.M.N. ¹H (300 MHz; CDCl₃ ; déplacements chimiques δ en ppm ; constantes de couplage J en Hz) : 0,98 (t, J = 7, 3H : CH₃ du propyle); 1,23 (s, 3H : CH₃); 1,33 (s, 3H : CH₃); 1,33 (s, 9H : C(CH₃)₃); 1,35 (mt, 1H : H en 7); de 1,55 à 1,80 et de 2,20 à 2,40 (2 mts, 1H chacun : CH₂ en 19); de 1,55 à 1,80 (mt, 2H : CH₂ central du propyle); 1,82 (s, 1H : OH en 1); 1,89 (s, 3H : CH₃); 2,12 et 2,40 (respectivement d large et dt, J = 15 et J = 15 et 4, 1H chacun : CH₂ en 6); 2,22 et de 2,20 à 2,40 (respectivement dd et mt, J = 16 et 9, 1H chacun : CH₂ en 14); 2,40 (s, 3H : COCH₃); 3,30 (mf, 1H: OH en 2'); 3,42 et 3,55 (2 mts, 1H chacun : OCH₂ du propyle) ; 4,04 et 4,32 (2 d, J = 8,5, 1H chacun : CH₂ en 20); 4,12 (d, J = 7,5, 1H : H en 3) ; 4,62 (mt, 1H : H en 2') ; 4,73 (d large, J = 4, 1H : H en 5) ; 4,80 (s, 1H : H en 10); 5,30 (d large, J = 10, 1H : H en 3') ; 5,37 (d, J = 10, 1H : CONH) ; 5,68 (d, J = 7,5, 1H : H en 2); 6,32 (t large, J = 9, 1H : H en 13); de 7,25 à 7,45 (mt, 5H : H aromatiques en 3'); 7,52 (t, J = 7,5, 2H : OCOC₆H₅ H en méta) ; 7,62 (t, J = 7,5, 1H : OCOC₆H₅ H en para) ; 8,12 (d, J = 7,5, 2H : OCOC₆H₅ H en ortho).

Le tert-butoxycarbonylamino-3 hydroxy-2 phényl-3 propionate-(2R,3S) d'acétoxy-4α benzoyloxy-2α époxy-5β,20 hydroxy-1β (propyl-1)oxy-10β oxo-9 trifluorométhanesulfonyloxy-7β taxène-11 yle-13α peut être préparé de la manière suivante :

Une solution de 340 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β (propyl-1)oxy-10β oxo-9 trifluorométhanesulfonyloxy-7β taxène-11 yle-13α dans 4,6 cm3 d'une solution 0,1N d'éthanol chlorhydrique est maintenue pendant 93 heures sous atmosphère d'argon à une température voisine de 0°C. Le mélange réactionnel est dilué avec 50 cm3 d'acétate d'éthyle, lavé avec 6 cm3 d'une solution aqueuse saturée en hydrogénocarbonate de sodium, 6 cm3 d'eau distillée puis 6 cm3 d'une solution aqueuse saturée en chlorure de sodium. La phase organique est séchée sur sulfate de magnésium, filtrée sur verre fritté puis concentrée à sec sous pression réduite (2,7 kPa) à une température voisine de 40°C. On obtient ainsi 348 mg d'une laque jaune que l'on purifie par chromatographie préparative sur couche mince : 6 plaques préparatives Merck, Kieselgel 60F254, épaisseur 1 mm, dépôt en solution dans le dichlorométhane, en éluant deux fois par un mélange méthanol-dichlorométhane (3-97 en volumes). Après élution de la zone correspondant au produit principal par un mélange méthanol-dichlorométhane (15-85 en volumes), filtration sur verre fritté, puis évaporation des solvants sous pression réduite (2,7 kPa) à 40°C pendant 2 heures. On obtient ainsi 61,4 mg de tert-butoxycarbonylamino-3 hydroxy-2 phényl-3 propionate-(2R,3S) d'acétoxy-4α benzoyloxy-2α époxy-5β,20 hydroxy-1β (propyl-1)oxy-10β oxo-9 trifluorométhanesulfonyloxy-7β taxène-11 yle-13α sous forme d'une meringue jaune dont les caractéristiques sont les suivantes :
- spectre de R.M.N. ¹H (400 MHz ; CDCl₃ ; déplacements chimiques δ en ppm ; constantes de couplage J en Hz) : 0,99 (t, J = 7, 3H : CH₃ du propyle) ; 1,24 (s, 3H : CH₃); 1,26 (s, 3H : CH₃); 1,39 (s, 9H : C(CH₃)₃); 1,64 (s, 1H : OH en 1) ; 1,69 (mt, 2H : CH₂ central du propyle) ; 1,87 (s, 3H : CH₃); 1,94 (s, 3H : CH₃); 2,26 et 2,83 (2 mts, 1H chacun : CH₂ en 6); 2,32 (d, J = 9, 2H : CH₂ en 14); 2,40 (s, 3H : COCH₃); 3,33 (d, J = 4, 1H : OH en 2'); 3,44 et 3,59 (2 mts, 1H chacun : OCH₂ du propyle) ; 3,97 (d, J = 7,5, 1H : H en 3) ; 4,19 et 4,35 (2 d, J = 8,5, 1H chacun : CH₂ en 20) ; 4,64 (mt, 1H : H en 2') ; 4,93 (d large, J = 10, 1H : H en 5) ; 5,17 (s, 1H : H en 10); 5,27 (d large, J = 10, 1H : H en 3'); 5,39 (d, J = 10, 1H : CONH) ; 5,46 (dd, J = 11 et 6,5, 1H : H en 7) ; 5,72 (d, J = 7,5, 1H : H en 2); 6,25 (t large, J = 9, 1H : H en 13); de 7,25 à 7,45 (mt, 5H : H aromatiques en 3'); 7,52 (t, J = 7,5, 2H : OCOC₆H₅ H en méta) ; 7,63 (t, J = 7,5, 1H : OCOC₆H₅ H en para) ; 8,12 (d, J = 7,5, 2H : OCOC₆H₅ H en ortho).

Le tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β (propyl-1)oxy-10β oxo-9 trifluorométhanesulfonyoxy-7β taxène-11 yle-13α peut être préparé de la manière suivante :

A une suspension de 204 mg d'acétoxy-4α benzoyloxy-2α époxy-5β,20 dihydroxy-1β,13α (propyl-1)oxy-10β oxo-9 trifluorométhanesulfonyloxy-7β taxène-11, 177 mg d'acide tert-butoxycarbonyl-3 (méthoxy-4 phényl)-2 phényl-4 oxazolidine-1,3 carboxylique-5-(2R,4S,5R) dans 2 cm3 de dichlorométhane, on ajoute successivement, à une température voisine de 20°C, 90 mg d'acide tert-butoxycarbonyl-3 (méthoxy-4 phényl)-2 phényl-4 oxazolidine-1,3 carboxylique-5-(2R,4S,5R), 60 mg de dicyclohexylcarbodiimide et 6,2 mg de N,N'-diméthylamino-4 pyridine. Après 65 heures à une température voisine de 20°C, on ajoute successivement, à une température voisine de 20°C, 113 mg de dicyclohexylcarbodiimide et 12,5 mg de N,N'-diméthylamino-4 pyridine. Après 2 heures à une température voisine de 20°C, le mélange réactionnel est purifié (dépôt direct sur la colonne) par chromatographie à pression atmosphérique sur 20 g de silice (0,063-0,2 mm) contenus dans une colonne de 2 cm de diamètre en éluant avec un mélange méthanol-dichlorométhane (4-96 en volumes) en recueillant des fractions de 10 cm3. Les fractions ne contenant que le produit cherché sont réunies et concentrées à sec sous pression réduite (0,27 kPa) à 40°C pendant 2 heures. On obtient ainsi 384 mg d'un solide jaune que l'on purifie par chromatographie préparative sur couche mince : 6 plaques préparatives Merck, Kieselgel 60F254, épaisseur 2 mm, dépôt en solution dans le dichlorométhane, en éluant deux fois par un mélange méthanol-dichlorométhane (3-97 en volumes). Après élution de la zone correspondant au produit principal par un mélange méthanol-dichlorométhane (15-85 en volumes), filtration sur verre fritté, puis évaporation des solvants sous pression réduite (2,7 kPa) à 40°C pendant 2 heures. On obtient ainsi 323,2 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β (propyl-1)oxy-10β oxo-9 trifluorométhanesulfonyloxy-7β taxène-11 yle-13α sous forme d'une meringue jaune dont les caractéristiques sont les suivantes :
- spectre de R.M.N. ¹H (300 MHz ; CDCl₃ ; déplacements chimiques δ en ppm ; constantes de couplage J en Hz) : 0,96 (t, J = 7, 3H : CH₃ du propyle); 1,08 (s, 9H : C(CH₃)₃); 1,19 (s, 6H : CH₃); 1,50 (s, 1H : OH en 1); 1,58 (s, 3H : CH₃); 1,65 (mt, 2H : CH₂ central du propyle) ; 1,80 (s, 3H : CH₃); 1,88 (mf, 3H : COCH₃); 2,04 et 2,12 (2 dd, J = 16 et 9, 1H chacun : CH₂ en 14); de 2,10 à 2,30 et 2,75 (2 mts, 1H chacun : CH₂ en 6); 3,35 et 3,50 (2 mts, 1H chacun : OCH₂ du propyle) ; 3,81 (s, 3H : ArOCH₃) ; 3,82 (d, J = 7,5, 1H : H en 3) ; 4,10 et 4,28 (2 d, J = 8,5, 1H chacun : CH₂ en 20) ; 4,57 (d, J = 4,5, 1H : H en 2') ; 4,80 (d large, J = 10, 1H : H en 5); 5,04 (s, 1H : H en 10); 5,38 (dd, J = 10,5 et 7, 1H : H en 7); 5,45 (mf, 1H : H 3'); 5,62 (d, J = 7,5, 1H : H en 2); 6,08 (t large, J = 9, 1H : H en 13); 6,40 (mf étalé, 1H : H en 5'); 6,92 (d, J = 8,5, 2H : H aromatiques en ortho du OCH₃); de 7,30 à 7,60 (mt, 7H : H aromatiques en 3' et H aromatiques en méta du OCH₃); 7,50 (t, J = 7,5, 2H : OCOC₆H₅ H en méta) ; 7,62 (t, J = 7,5, 1H : OCOC₆H₅ H en para) ; 8,02 (d, J = 7,5, 2H : OCOC₆H₅ H en ortho).

L'acétoxy-4α benzoyloxy-2α époxy-5β,20 dihydroxy-1β,13α (propyl-1)oxy-10β oxo-9 trifluorométhanesulfonyloxy-7β taxène-11 peut être préparé de la manière suivante :

A une suspension de 200 mg d'acétoxy-4α benzoyloxy-2α époxy-5β,20 trihydroxy-1β,7β,13α (propyl-1)oxy-10β oxo-9 taxène-11 dans 2 cm3 de dichlorométhane anhydre et 0,14 cm3 de pyridine anhydre, refroidie à une température voisine de 0°C, maintenue sous atmosphère d'argon, on ajoute goutte à goutte 0,145 cm3 d'anhydride trifluorométhanesulfonique. Le mélange réactionnel est agité à une température voisine de 0°C pendant 55 minutes, puis dilué avec 2 cm3 d'un mélange méthanol-dichlorométhane (10-90 en volumes), concentré à sec sous pression réduite (2,7 kPa) à une température voisine de 30°C. Le résidu brut obtenu est purifié par chromatographie à pression atmosphérique sur 20 g de silice (0,063-0,2 mm) contenus dans une colonne de 2,5 cm de diamètre en éluant avec du dichlorométhane, en recueillant des fractions de 10 cm3. Les fractions ne contenant que le produit cherché sont réunies et concentrées à sec sous pression réduite (2,7 kPa) à 40°C pendant 2 heures. On obtient ainsi 204 mg d'acétoxy-4α benzoyloxy-2α époxy-5β,20 dihydroxy-1β,13α (propyl-1)oxy-10β oxo-9 trifluorométhanesulfonyloxy-7β taxène-11 sous forme d'une meringue brune.

L'acétoxy-4α benzoyloxy-2α époxy-5β,20 trihydroxy-1β,7β,13α (propyl-1) oxy-10β oxo-9 taxène-11 (ou (propyl-1)oxy-10β désacétoxy-10 baccatine III) peut être préparé de la manière suivante :

A une solution de 591 mg d'acétoxy-4α benzoyloxy-2α époxy-5β,20 hydroxy-1β(propyl-1)oxy-10β oxo-9 bistriéthylsilyloxy-7β,13α taxène-11 dans 6 cm3 de dichlorométhane, maintenue sous atmosphère d'argon, à une température voisine de 20°C, on ajoute 9 cm3 de complexe fluorure d'hydrogène-triéthylamine (3HF.Et₃N). Après 21 heures à une température voisine de 20°C, le mélange réactionnel est dilué avec 40 cm3 de dichlorométhane et versé sur une suspension de 40 cm3 d'une solution aqueuse sursaturée en hydrogénocarbonate de sodium, maintenue à une température voisine de 0°C. Après dilution avec 10 cm3 d'eau distillée et décantation, la phase aqueuse est réextraite avec deux fois 20 cm3 de diéthyléther. Les phases organiques sont rassemblées, lavées avec 20 cm3 d'eau distillée, 20 cm3 d'une solution aqueuse saturée en chlorure de sodium, séchées sur sulfate de magnésium, filtrées sur sulfate de magnésium et concentrées à sec sous pression réduite (2,7 kPa) à une température voisine de 40°C. On obtient ainsi 370 mg d'une meringue jaune pâle que l'on purifie par chromatographie à pression atmosphérique sur 35 g de silice (0,063-0,2 mm) contenus dans une colonne de 2,5 cm de diamètre en éluant avec un mélange méthanol-dichlorométhane (2-98 en volumes) en recueillant des fractions de 15 cm3. Les fractions ne contenant que le produit cherché sont réunies et concentrées à sec sous pression réduite (2,7 kPa) à 40°C pendant 2 heures. On obtient ainsi 236,2 mg d'acétoxy-4α benzoyloxy-2α époxy-5β,20 trihydroxy-1β,7β,13α (propyl-1) oxy-10β oxo-9 taxène-11 sous forme d'un solide blanc dont les caractéristiques sont les suivantes :
- spectre de R.M.N. ¹H (400 MHz; CDCl₃ ; déplacements chimiques δ en ppm ; constantes de couplage J en Hz) : 1,08 (s, 3H : CH₃); 1,19 (s, 3H : CH₃); 1,29 (t, J = 7,5, 3H : CH₃ éthyle) ; 1,38 (d, J = 9, 1H : OH en 7); 1,59 (s, 1H : OH en 1); 1,69 (s, 3H : CH₃); 1,82 et 2,62 (2 mts, 1H chacun : CH₂ en 6); 2,02 (d, J = 5, 1H : OH en 13); 2,08 (s, 3H : CH₃); 2,30 (s, 3H : COCH₃); 2,32 (d, J = 9, 2H : CH₂ en 14); 3,56 et 3,67 (2 mts, 1H chacun : OCH₂ éthyle) ; 3,98 (d, J = 7, 1H : H en 3) ; 4,18 et 4,33 (2 d, J = 8,5, 1H chacun : CH₂ en 20) ; 4,30 (mt, 1H : H en 7) ; 4,90 (mt, 1H : H en 13) ; 4,99 (dd, J = 10 et 1,5, 1H : H en 5) ; 5,05 (s, 1H : H en 10) ; 5,66 (d, J = 7, 1H : H en 2) ; 7,49 (t, J = 7,5, 2H : OCOC₆H₅ H en méta) ; 7,63 (t, J = 7,5, 1H : OCOC₆H₅ H en para) ; 8,12 (d, J = 7,5, 2H : OCOC₆H₅ H en ortho).

L'acétoxy-4α benzoyloxy-2α époxy-5β,20 hydroxy-1β (propyl-1) oxy-10β oxo-9 bistriéthylsilyloxy-7β,13α taxène-11 (ou (propyl-1) oxy-10β désacétoxy-10 bistriéthylsilyl-7,13 baccatine III) peut être préparé de la manière suivante :

A une solution de 1 g d'acétoxy-4α benzoyloxy-2α époxy-5β,20 dihydroxy-1β,10β oxo-9 bistriéthylsilyloxy-7β,13α taxène-11 dans 3 cm3 d'iodoéthane et 4 cm3 de diméthylformamide, maintenue sous atmosphère d'argon, à une température voisine de 20°C, on ajoute par portions 93 mg d'hydrure de sodium à 50 % en poids dans l'huile de vaseline. La solution est maintenue sous agitation 17 heures à une température voisine de 20°C, puis on ajoute par portions 93 mg d'hydrure de sodium à 50 % en poids dans l'huile de vaseline. Après 50 minutes à une température voisine de 20°C le mélange réactionnel est dilué avec 100 cm3 d'acétate d'éthyle, 10 cm3 d'une solution aqueuse saturée en chlorure d'ammonium. La phase organique décantée est lavée avec six fois 10 cm3 d'eau distillée, puis 10 cm3 d'une solution aqueuse saturée en chlorure de sodium, séchée sur sulfate de magnésium, filtrée sur verre fritté, et concentrée à sec sous pression réduite (2,7 kPa) à une température voisine de 40°C. On obtient ainsi 1,2 g d'une meringue jaune que l'on purifie par chromatographie à pression atmosphérique sur 150 g de silice (0,063-0,2 mm) contenus dans une colonne de 3,5 cm de diamètre en éluant avec un mélange acétate d'éthyle-dichlorométhane (2-98 puis 5-95 en volumes) en recueillant des fractions de 15 cm3. Les fractions ne contenant que les produits cherchés sont réunies et concentrées à sec sous pression réduite (0,27 kPa) à 40°C pendant 2 heures. On obtient ainsi 379,2 mg d'acétoxy-4α benzoyloxy-2α époxy-5β,20 dihydroxy-1β,10β oxo-9 bistriéthylsilyloxy-7β,13α taxène-11 sous forme d'une meringue jaune pâle et 430 mg d'acétoxy-4α benzoyloxy-2α époxy-5β,20 hydroxy-1β (propyl-1) oxy-10β oxo-9 bistriéthylsilyloxy-7β,13α taxène-11 sous forme d'une meringue blanche dont les caractéristiques sont les suivantes :
- spectre de R.M.N. ¹H (400 MHz; CDCl₃; déplacements chimiques δ en ppm ; constantes de couplage J en Hz) : 0,57 et 0,70 (2 mts, 6H chacun : CH₂ de l'éthyle) ; 0,97 et 1,03 (2 t, J = 7,5, 9H chacun : CH₃ de l'éthyle) ; 1,13 (s, 3H : CH₃); 1,20 (s, 3H : CH₃); 1,29 (t, J = 7,5, 3H : CH₃ de l'éthoxy en 10); 1,58 (s, 1H : OH en 1); 1,66 (s, 3H : CH₃); 1,89 et 2,58 (2 mts, 1H chacun : CH₂ 6) ; 2,03 (s, 3H : CH₃); 2,13 et 2,23 (2 dd, J =16 et 9, 1H chacun : CH₂ en 14) ; 2,30 (s, 3H: COCH₃); 3,53 (mt, 2H : CH₂ de l'éthoxy en 10) ; 3,84 (d, J = 7, 1H : H en 3) ; 4,15 et 4,30 (2 d, J = 8,5, 1H chacun : CH₂ en 20) ; 4,43 (dd, J = 11 et 6,5, 1H : H en 7); de 4,90 à 5,00 (mt, 2H : H en 13 et H en 5) ; 5,01 (s, 1H : H en 10); 5,61 (d, J = 7, 1H : H en 2) ; 7,48 (t, J = 7,5, 2H : OCOC₆H₅ H en méta) ; 7,61 (t, J = 7,5, 1H : OCOC₆H₅ H en para) ; 8,10 (d, J = 7,5, 2H : OCOC₆H₅ H en ortho).

Les nouveaux produits de formule générale (I) dans laquelle Z représente un radical de formule générale (II) manifestent une activité inhibitrice significative de la prolifération cellulaire anormale et possèdent des propriétés thérapeutiques permettant le traitement de malades ayant des conditions pathologiques associées à une prolifération cellulaire anormale. Les conditions pathologiques incluent la prolifération cellulaire anormale de cellules malignes ou non malignes de divers tissus et/ou organes, comprenant, de manière non limitative, les tissus musculaires, osseux ou conjonctifs, la peau, le cerveau, les poumons, les organes sexuels, les systèmes lymphatiques ou rénaux, les cellules mammaires ou sanguines, le foie, l'appareil digestif, le pancréas et les glandes thyroïdes ou adrénales. Ces conditions pathologiques peuvent inclure également le psoriasis, les tumeurs solides, les cancers de l'ovaire, du sein, du cerveau, de la prostate, du colon, de l'estomac, du rein ou des testicules, le sarcome de Kaposi, le cholangiocarcinome, le choriocarcinome, le neuroblastome, la tumeur de Wilms, la maladie de Hodgkin, les mélanomes, les myélomes multiples, les leucémies lymphocytaires chroniques, les lymphomes granulocytaires aigus ou chroniques. Les nouveaux produits selon l'invention sont particulièrement utiles pour le traitement du cancer de l'ovaire. Les produits selon l'invention peuvent être utilisés pour prévenir ou retarder l'apparition ou la réapparition des conditions pathologiques ou pour traiter ces conditions pathologiques.

Les produits selon l'invention peuvent être administrés à un malade selon différentes formes adaptées à la voie d'administration choisie qui, de préférence, est la voie parentérale. L'administration par voie parentérale comprend les administrations intraveineuse, intrapéritonéale, intramusculaire ou sous-cutanée. Plus particulièrement préférée est l'administration intrapéritonéale ou intraveineuse.

La présente invention comprend également les compositions pharmaceutiques qui contiennent au moins un produit de formule générale (I) en une quantité suffisante adaptée à l'emploi en thérapeutique humaine ou vétérinaire. Les compositions peuvent être préparées selon les méthodes habituelles en utilisant un ou plusieurs adjuvants, supports ou excipients pharmaceutiquement acceptables. Les supports convenables incluent les diluants, les milieux aqueux stériles et divers solvants non toxiques. De préférence les compositions se présentent sous forme de solutions ou de suspensions aqueuses, de solutions injectables qui peuvent contenir des agents émulsifiants, des colorants, des préservatifs ou des stabilisants.

Le choix des adjuvants ou excipients peut être déterminé par la solubilité et les propriétés chimiques du produit, le mode particulier d'administration et les bonnes pratiques pharmaceutiques.

Pour l'administration parentérale, on utilise des solutions ou des suspensions stériles aqueuses ou non aqueuses. Pour la préparation de solutions ou de suspensions non aqueuses peuvent être utilisés des huiles végétales naturelles telle que l'huile d'olive, l'huile de sésame ou l'huile de paraffine ou les esters organiques injectables tel que l'oléate d'éthyle. Les solutions stériles aqueuses peuvent être constituées d'une solution d'un sel pharmaceutiquement acceptable en solution dans de l'eau. Les solutions aqueuses conviennent pour l'administration intraveineuse dans la mesure où le pH est convenablement ajusté et où l'isotonicité est réalisée, par exemple, par une quantité suffisante de chlorure de sodium ou de glucose. La stérilisation peut être réalisée par chauffage ou par tout autre moyen qui n'altère pas la composition.

Il est bien entendu que tous les produits entrant dans les compositions selon l'invention doivent être purs et non toxiques pour les quantités utilisées.

Les compositions peuvent contenir au moins 0,01 % de produit thérapeutiquement actif. La quantité de produit actif dans une composition est telle qu'une posologie convenable puisse être prescrite. De préférence, les compositions sont préparées de telle façon qu'une dose unitaire contienne de 0,01 à 1000 mg environ de produit actif pour l'administration par voie parentérale.

Le traitement thérapeutique peut être effectué concurremment avec d'autres traitements thérapeutiques incluant des médicaments antinéoplastiques, des anticorps monoclonaux, des thérapies immunologiques ou des radiothérapies ou des modificateurs des réponses biologiques. Les modificateurs des réponses incluent, de manière non limitative, les lymphokines et les cytokines telles que les interleukines, les interférons (α, β ou δ) et le TNF. D'autres agents chimiothérapeutiques utiles dans le traitement des désordres dus à la prolifération anormale des cellules incluent, de manière non limitative, les agents alkylants tels que les moutardes à l'azote comme la mechloretamine, le cyclophosphamide, le melphalan et le chlorambucil, des sulfonates d'alkyle comme le busulfan, les nitrosourées comme la carmustine, la lomustine, la sémustine et la streptozocine, les triazènes comme la dacarbazine, les antimétabolites comme les analogues de l'acide folique tel que le méthotrexate, les analogues de pyrimidine comme le fluorouracil et la cytarabine, des analogues de purines comme la mercaptopurine et la thioguanine, des produits naturels tels que les alcaloïdes de vinca comme la vinblastine, la vincristine et la vendésine, des épipodophyllotoxines comme l'étoposide et le teniposide, des antibiotiques comme la dactinomycine, la daunorubicine, la doxorubicine, la bléomycine, la plicamycine et la mitomycine, des enzymes comme la L-asparaginase, des agents divers comme les complexes de coordination du platine tel que le cisplatine, les urées substituées tel que l'hydroxyurée, les dérivés de méthylhydrazine comme la procarbazine, les suppresseurs adrénocoticoïques comme le mitotane et l'aminoglutéthymide, les hormones et les antagonistes comme les adrénocorticostéroïdes comme la prednisone, les progestines comme le caproate d'hydroxyprogestérone, l'acétate de méthoxyprogestérone et l'acétate de megestrol, les oestrogènes comme le diéthylstilbestrol et l'éthynylestradiol, les antioestrogène comme le tamoxifène, les androgènes comme le propionate de testostérone et la fluoxymesterone.

Les doses utilisées pour mettre en oeuvre les méthodes selon l'invention sont celles qui permettent un traitement prophylactique ou un maximum de réponse thérapeutique. Les doses varient selon la forme d'administration, le produit particulier sélectionné et les caractéristiques propres du sujet à traiter. En général, les doses sont celles qui sont thérapeutiquement efficaces pour le traitement des désordres dus à une prolifération cellulaire anormale. Les produits selon l'invention peuvent être administrés aussi souvent que nécessaire pour obtenir l'effet thérapeutique désiré. Certains malades peuvent répondre rapidement à des doses relativement fortes ou faibles puis avoir besoin de doses d'entretien faibles ou nulles. Généralement, de faibles doses seront utilisées au début du traitement et, si nécessaire, des doses de plus en plus fortes seront administrées jusqu'à l'obtention d'un effet optimum. Pour d'autres malades il peut être nécessaire d'administrer des doses d'entretien 1 à 8 fois par jour, de préférence 1 à 4 fois, selon les besoins physiologiques du malade considéré. Il est aussi possible que pour certains malades il soit nécessaire de n'utiliser qu'une à deux administrations journalières.

Chez l'homme, les doses sont généralement comprises entre 0,01 et 200 mg/kg. Par voie intrapéritonéale, les doses seront en général comprises entre 0,1 et 100 mg/kg et, de préférence entre 0,5 et 50 mg/kg et, encore plus spécifiquement entre 1 et 10 mg/kg. Par voie intraveineuse, les doses sont généralement comprises entre 0,1 et 50 mg/kg et, de préférence entre 0,1 et 5 mg/kg et, encore plus spécifiquement entre 1 et 2 mg/kg. Il est entendu que, pour choisir le dosage le plus approprié, devront être pris en compte la voie d'administration, le poids du malade, son état de santé général, son âge et tous les facteurs qui peuvent influer sur l'efficacité du traitement.

L'exemple suivant illustre une composition selon l'invention.

### EXEMPLE

On dissout 40 mg du produit obtenu à l'exemple 1 dans 1 cm3 d'Emulphor EL 620 et 1 cm3 d'éthanol puis la solution est diluée par addition de 18 cm3 de sérum physiologique.

La composition est administrée par perfusion pendant 1 heure par introduction dans du soluté physiologique.

## Revendications

1. Taxoïdes de formule générale : 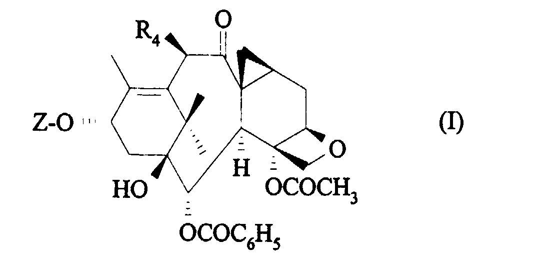 dans laquelle :
Z représente un atome d'hydrogène ou un radical de formule générale : 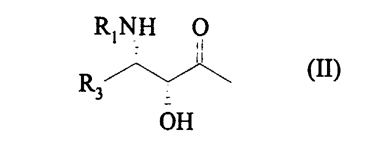 dans laquelle :
R₁ représente un radical benzoyle éventuellement substitué par un ou plusieurs atomes ou radicaux, identiques ou différents, choisis parmi les atomes d'halogène et les radicaux alcoyles contenant 1 à 4 atomes de carbone, alcoxy contenant 1 à 4 atomes de carbone ou trifluorométhyle, thénoyle ou furoyle ou un radical R₂-O-CO- dans lequel R₂ représente :
- un radical alcoyle contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone, bicycloalcoyle contenant 7 à 10 atomes de carbone, ces radicaux étant éventuellement substitués par un ou plusieurs substituants choisis parmi les atomes d'halogène et les radicaux hydroxy, alcoxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle (éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone), cyano, carboxy ou alcoxycarbonyle dont la partie alcoyle contient 1 à 4 atomes de carbone,
- un radical phényle ou α- ou β-naphtyle éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone ou un radical hétérocyclique aromatique à 5 chaînons choisi de préférence parmi les radicaux furyle et thiényle,
- ou un radical hétérocyclyle saturé contenant 4 à 6 atomes de carbone éventuellement substitué par un ou plusieurs radicaux alcoyles contenant 1 à 4 atomes de carbone,
R₃ représente un radical
- alcoyle droit ou ramifié contenant 1 à 8 atomes de carbone,
- alcényle droit ou ramifié contenant 2 à 8 atomes de carbone,
- alcynyle droit ou ramifié contenant 2 à 8 atomes de carbone,
- cycloalcoyle contenant 3 à 6 atomes de carbone,
- phényle ou α- ou β-naphtyle
éventuellement substitué par un ou plusieurs atomes ou radicaux choisis parmi les atomes d'halogène et les radicaux alcoyles, alcényles, alcynyles, aryles, aralcoyles, alcoxy, alcoylthio, aryloxy, arylthio, hydroxy, hydroxyalcoyle, mercapto, formyle, acyle, acylamino, aroylamino, alcoxycarbonyl-amino, amino, alcoylamino, dialcoylamino, carboxy, alcoxycarbonyle, carbamoyle, alcoylcarbamoyle, dialcoylcarbamoyle, cyano, nitro et trifluorométhyle,
- ou un hétérocycle aromatique ayant 5 chaînons et contenant un ou plusieurs hétéroatomes, identiques ou différents, choisis parmi les atomes d'azote, d'oxygène ou de soufre et éventuellement substitué par un ou plusieurs substituants, identiques ou différents, choisis parmi les atomes d'halogène et les radicaux alcoyles, aryles, amino, alcoylamino, dialcoylamino, alcoxycarbonylamino, acyle, arylcarbonyle, cyano, carboxy, carbamoyle, alcoylcarbamoyle, dialcoylcarbamoyle ou alcoxycarbonyle, étant entendu que, dans les substituants des radicaux phényle, α- ou β-naphtyle et hétérocyclyles aromatiques, les radicaux alcoyles et les portions alcoyles des autres radicaux contiennent 1 à 4 atomes de carbone et que les radicaux alcényles et alcynyles contiennent 2 à 8 atomes de carbone et que les radicaux aryles sont des radicaux phényles ou α- ou β-naphtyles,
R₄ représente un radical
- alcoxy contenant 1 à 6 atomes de carbone en chaîne droite ou ramifiée,
- alcényloxy contenant 3 à 6 atomes de carbone en chaîne droite ou ramifiée,
- alcynyloxy contenant 3 à 6 atomes de carbone en chaîne droite ou ramifiée,
- cycloalcoyloxy contenant 3 à 6 atomes de carbone,
- cycloalcényloxy contenant 3 à 6 atomes de carbone
éventuellement substitué par un ou plusieurs atomes d'halogène ou par un radical alcoxy contenant 1 à 4 atomes de carbone, alcoylthio contenant 1 à 4 atomes de carbone, ou un radical carboxy, alcoyloxycarbonyle dont la partie alcoyle contient 1 à 4 atomes de carbone, cyano, carbamoyle, N-alcoylcarbamoyle ou N,N-dialcoylcarbamoyle dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote éventuellement substitué par un radical alcoyle contenant 1 à 4 atomes de carbone, ou un radical phényle ou un radical phénylalcoyle dont la partie alcoyle contient 1 à 4 atomes de carbone.

2. Taxoïdes selon la revendication 1 pour lesquels Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical alcoyle contenant 1 à 6 atomes de carbone, alcényle contenant 2 à 6 atomes de carbone, cycloalcoyle contenant 3 à 6 atomes de carbone, phényle éventuellement substitué par un ou plusieurs atomes ou radicaux, identiques ou différents choisis parmi les atomes d'halogène et les radicaux alcoyles, alcoxy, dialcoylamino, acylamino, alcoxycarbonylamino ou trifluorométhyle ou un radical furyle-2 ou -3, thiényle-2 ou -3 ou thiazolyle-2, -4 ou -5 et R₄ représente un radical alcoyloxy droit ou ramifié contenant 1 à 6 atomes de carbone.

3. Taxoïdes selon la revendication 1 pour lesquels Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical isobutyle, isobutényle, butényle, cyclohexyle, phényle, furyle-2, furyle-3, thiényle-2, thiényle-3, thiazolyle-2, thiazolyle-4 ou thiazolyle-5, R₄ représente un radical méthoxy, éthoxy ou propoxy.

4. Procédé de préparation d'un produit selon l'une des revendications 1, 2 ou 3 caractérisé en ce que estérifie un produit de formule générale : 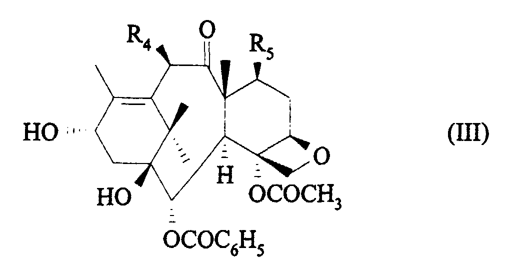 dans laquelle R₄ est défini comme dans l'une des revendications 1, 2 ou 3 et R₅ représente un radical trifluorométhanesulfonyloxy ou forme une liaison avec l'atome de carbone du radical méthyle en α de façon à former un cycle cyclopropane, au moyen d'un acide de formule générale : 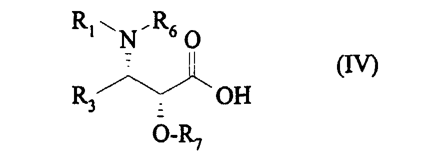 dans laquelle R₁ et R₃ sont définis comme dans l'une des revendications 1, 2 ou 3, ou bien R₆ représente un atome d'hydrogène et R₇ représente un groupement protecteur de la fonction hydroxy, et ou bien R₆ et R₇ forment ensemble un hétérocycle saturé à 5 ou 6 chaînons, ou d'un dérivé de cet acide pour obtenir un ester de formule générale : 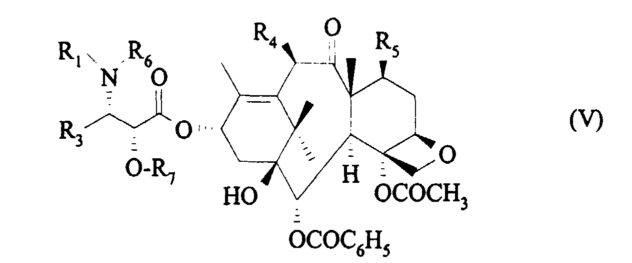 dans laquelle R₁, R₃, R₄, R₅, R₆ et R₇ sont définis comme précédemment, puis remplace les groupements protecteurs représentés par R₇ et/ou R₆ et R₇ par des atomes d'hydrogène et, lorsque R₅ représente un radical trifluorométhanesulfonyloxy, élimine ce radical de façon à former un cycle cyclopropane avec l'atome de carbone du radical méthyle en α.

5. Procédé selon la revendication 4 caractérisé en ce que l'on effectue l'estérification au moyen d'un acide de formule générale (IV) en présence d'un agent de condensation et d'un agent d'activation dans un solvant organique à une température comprise entre -10 et 90°C.

6. Procédé selon la revendication 4 caractérisé en ce que l'on effectue l'estérification au moyen d'un acide de formule générale (IV) sous forme d'anhydride symétrique en opérant en présence d'un agent d'activation dans un solvant organique à une température comprise entre 0 et 90°C.

7. Procédé selon la revendication 4 caractérisé en ce que l'on effectue l'estérification en utilisant l'acide de formule générale (IV) sous forme d'halogénure ou sous forme d'anhydride mixte avec un acide aliphatique ou aromatique, éventuellement préparé in situ, en présence d'une base en opérant dans un solvant organique à une température comprise entre 0 et 80°C.

8. Procédé selon la revendication 4 caractérisé en ce que l'on remplace les groupements protecteurs R₇ et/ou R₆ et R₇ par des atomes d'hydrogène en opérant de la manière suivante :
1) lorsque R₆ représente un atome d'hydrogène et R₇ représente un groupement protecteur de la fonction hydroxy, on remplace les groupements protecteurs par des atomes d'hydrogène s'effectue au moyen d'un acide minéral ou organique utilisé seul ou en mélange en opérant dans un solvant organique choisi parmi les alcools, les éthers, les esters, les hydrocarbures aliphatiques, les hydrocarbures aliphatiques halogénés, les hydrocarbures aromatiques ou les nitriles à une température comprise entre -10 et 60°C,
2) lorsque R₆ et R₇ forment ensemble un hétérocycle saturé à 5 ou 6 chaînons et plus particulièrement un cycle oxazolidine de formule générale : 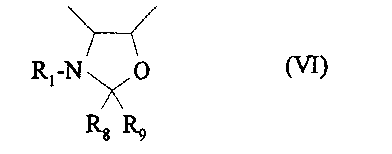 dans laquelle R₁ est défini comme dans l'une des revendications 1, 2 ou 3, R₈ et R₉, identiques ou différents, représentent un atome d'hydrogène ou un radical alcoyle contenant 1 à 4 atomes de carbone, ou un radical aralcoyle dont la partie alcoyle contient 1 à 4 atomes de carbone et la partie aryle représente un radical phényle éventuellement substitué par un ou plusieurs radicaux alcoxy contenant 1 à 4 atomes de carbone, ou un radical aryle représentant un radical phényle éventuellement substitué par un ou plusieurs radicaux alcoxy contenant 1 à 4 atomes de carbone, ou bien R₈ représente un radical alcoxy contenant 1 à 4 atomes de carbone ou un radical trihalométhyle ou un radical phényle substitué par un radical trihalométhyle et R₉ représente un atome d'hydrogène, ou bien R₈ et R₉ forment ensemble avec l'atome de carbone auquel ils sont liés un cycle ayant 4 à 7 chaînons, on remplace le groupement protecteur formé par R₆ et R₇ par des atomes d'hydrogène en opérant, selon les significations de R₁, R₈ et R₉, de la manière suivante :
a) lorsque R₁ représente un radical tert-butoxycarbonyle, R₈ et R₉, identiques ou différents, représentent un radical alcoyle ou un radical aralcoyle ou aryle, ou bien R₈ représente un radical trihalométhyle ou un radical phényle substitué par un radical trihalométhyle, et R₉ représente un atome d'hydrogène, ou bien R₈ et R₉ forment ensemble un cycle ayant de 4 à 7 chaînons, on traite de l'ester de formule générale (V) par un acide minéral ou organique éventuellement dans un solvant organique tel qu'un alcool pour obtenir un produit de formule générale : 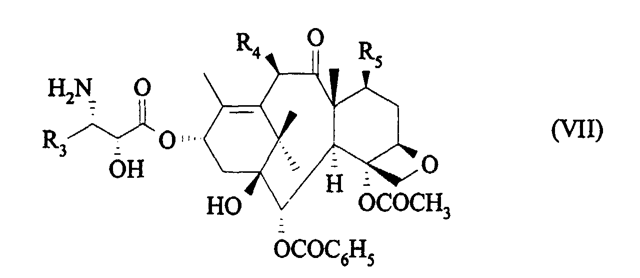 dans laquelle R₃, R₄ et R₅ sont définis dans l'une des revendications 1, 2 ou 3, que l'on acyle au moyen de chlorure de benzoyle dans lequel le noyau phényle est éventuellemnt substitué, de chlorure de thénoyle, de chlorure de furoyle ou d'un produit de formule générale :
R₂-O-CO-X (VIII)
dans laquelle R₂ est défini comme précédemment et X représente un atome d'halogène ou un reste -O-R₂ ou -O-CO-O-R₂, pour obtenir un produit de formule générale (I) dans laquelle Z représente un radical de formule générale (II).
b) lorsque R₁ représente un radical benzoyle éventuellement substitué, thénoyle ou furoyle ou un radical R₂O-CO- dans lequel R₂ est défini comme précédemment, R₈ représente un atome d'hydrogène ou un radical alcoxy contenant 1 à 4 atomes de carbone ou un radical phényle substitué par un ou plusieurs radicaux alcoxy contenant 1 à 4 atomes de carbone et R₉ représente un atome d'hydrogène, on remplace le groupement protecteur formé par R₆ et R₇ par des atomes d'hydrogène en présence d'un acide minéral ou organique utilisé seul ou en mélange en quantité stoechiométrique ou catalytique, en opérant dans un solvant organique choisi parmi les alcools, les éthers, les esters, les hydrocarbures aliphatiques, les hydrocarbures aliphatiques halogénés et les hydrocarbures aromatiques à une température comprise entre -10 et 60°C, de préférence entre 15 et 30°C.

9. Procédé selon la revendication 4 caractérisé en ce que l'on élimine le radical R₅ représentant un radical trifluorométhanesulfonyloxy pour former une liaison avec l'atome de carabone du radical méthyle en a au moyen d'un halogénure de métal alcalin ou d'un azoture de métal alcalin ou d'un sel d'ammonium en opérant dans un solvant organique choisi parmi les éthers, les nitriles ou les esters aliphatiques seul ou en mélange à une température comprise entre 20°C et la température d'ébullition du mélange réactionnel.

10. Procédé de préparation d'un nouveau taxoïde de formule générale : 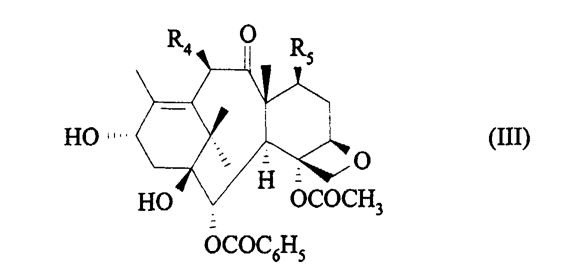 dans laquelle R₄ est défini comme dans l'une des revendications 1, 2 ou 3 et R₅ représente un radical trifluorométhanesulfonyloxy ou forme une liaison avec l'atome de carbone du radical méthyle en α caractérisé en ce que l'on traite la 10-désacétyl-baccatine III de formule : 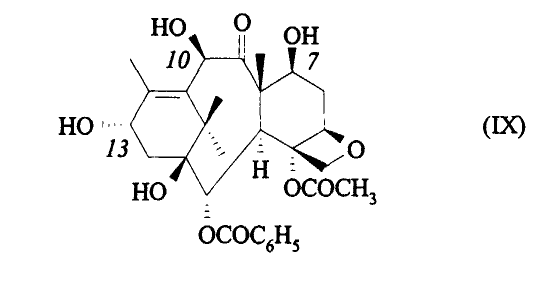 par un halogénure de silyle de formule générale :
(R')₃-Si-Hal (X)
dans laquelle les symboles R', identiques ou différents, représentent un radical alcoyle contenant 1 à 4 atomes de carbone éventuellement substitué par un radical phényle, ou un radical phényle pour obtenir un produit de formule générale : 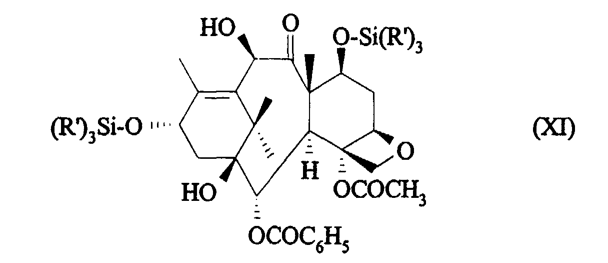 dans laquelle R' est défini comme précédemment, que l'on traite par un produit de formule générale :
R'₄-X₁ (XII)
dans laquelle R'₄ est tel que R'₄-O est identique à R₄ comme dans l'une des revendications 1, 2 ou 3 et X₁ représente un atome d'halogène ou un reste d'ester réactif tel qu'un reste d'ester sulfurique ou sulfonique pour obtenir un produit de formule générale : 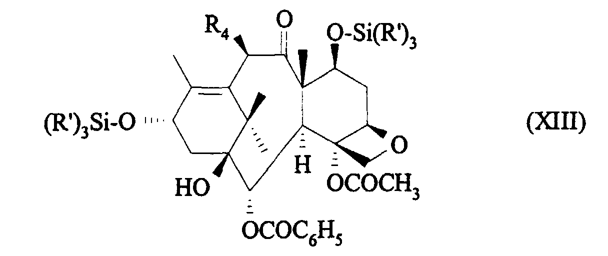 dans laquelle R' et R₄ sont définis comme précédemment, dont on remplace les groupements protecteurs silylés par des atomes d'hydrogène pour obtenir un produit de formule générale : 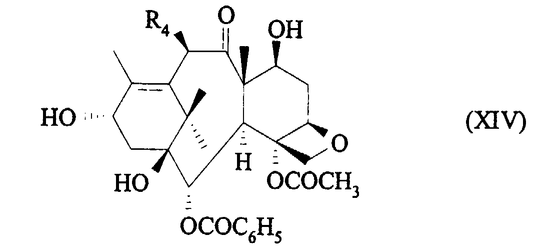 dans laquelle R₄ est défini comme précédemment, que l'on traite par un dérivé de l'acide trifluorométhanesulfonique pour obtenir un produit de formule générale (III) dans laquelle R₅ représente un radical trifluorométhanesulfonyloxy, que l'on traite éventuellement par un halogénure de métal alcalin ou un azoture de métal alcalin ou un sel d'ammonium en opérant dans un solvant organique choisi parmi les éthers et les nitriles et les esters aliphatiques seul ou en mélange à une température comprise entre 20°C et la température d'ébullition du mélange réactionnel, pour obtenir un produit de formule générale (III) dans laquelle R₅ forme une liaison avec l'atome de carbone du radical méthyle en α.

11. Le tert-butoxycarbonylamino-3 hydroxy-2 phényl-3 propionate-(2R,3S) d'acétoxy-4α benzoyloxy-2α époxy-5β,20 hydroxy-1β méthoxy-10β méthylène-7β,8β oxo-9 nor-19 taxène-11 yle-13α.

12. Le tert-butoxycarbonylamino-3 hydroxy-2 phényl-3 propionate-(2R,3S) d'acétoxy-4α benzoyloxy-2α époxy-5β,20 hydroxy-1β éthoxy-10β méthylène-7β,8β oxo-9 nor-19 taxène-11 yle-13α.

13. Le tert-butoxycarbonylamino-3 hydroxy-2 phényl-3 propionate-(2R,3S) d'acétoxy-4α benzoyloxy-2α époxy-5β,20 hydroxy-1β (propyl-1)oxy-10β méthylène-7β,8β oxo-9 nor-19 taxène-11 yle-13α

14. Composition pharmaceutique caractérisée en ce qu'elle contient au moins un produit selon l'une des revendications 1, 2 ou 3 pour lequel Z représente un radical de formule générale (II) en association avec un ou plusieurs diluants ou adjuvants pharmaceutiquement acceptables et éventuellement un ou plusieurs composés compatibles et pharmacologiquement actifs.

15. Composition pharmaceutique caractérisée en ce qu'elle contient au moins un produit selon la revendication 11 en association avec un ou plusieurs diluants ou adjuvants pharmaceutiquement acceptables et éventuellement un ou plusieurs composés compatibles et pharmacologiquement actifs.

16. Composition pharmaceutique caractérisée en ce qu'elle contient au moins un produit selon la revendication 12 en association avec un ou plusieurs diluants ou adjuvants pharmaceutiquement acceptables et éventuellement un ou plusieurs composés compatibles et pharmacologiquement actifs.

17. Composition pharmaceutique caractérisée en ce qu'elle contient au moins un produit selon la revendication 13 en association avec un ou plusieurs diluants ou adjuvants pharmaceutiquement acceptables et éventuellement un ou plusieurs composés compatibles et pharmacologiquement actifs.

## Patentansprüche

1. Taxoide der allgemeinen Formel: 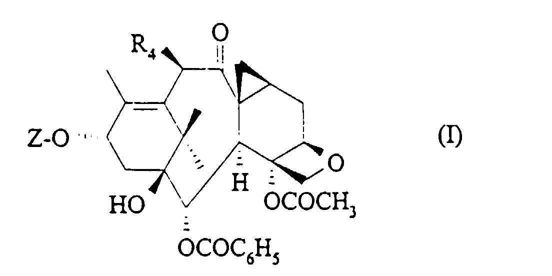 in der:
Z ein Wasserstoffatom oder einen Rest der allgemeinen Formel: 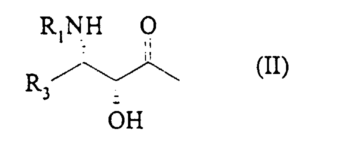 darstellt, in der:
R₁ einen Benzoylrest, der gegebenenfalls mit einem oder mehreren, gleichen oder verschiedenen Atomen oder Resten substituiert ist, die ausgewählt sind unter den Halogenatomen und den Alkylresten mit 1 bis 4 Kohlenstoffatomen, den Alkoxyresten mit 1 bis 4 Kohlenstoffatomen oder dem Trifluormethyl-, Thenoyl- oder Furoylrest, oder einen Rest R₂-O-CO- darstellt, in dem R₂ darstellt:
- einen Alkylrest mit 1 bis 8 Kohlenstoffatomen, Alkenylrest mit 2 bis 8 Kohlenstoffatomen, Alkinylrest mit 3 bis 8 Kohlenstoffatomen, Cycloalkylrest mit 3 bis 6 Kohlenstoffatomen, Cycloalkenylrest mit 4 bis 6 Kohlenstoffatomen, Bicycloalkylrest mit 7 bis 10 Kohlenstoffatomen, wobei diese Reste gegebenenfalls mit einem oder mehreren Substituenten substituiert sind, die ausgewählt sind unter den Halogenatomen und dem Hydroxyrest, den Alkoxyresten mit 1 bis 4 Kohlenstoffatomen, den Dialkylaminoresten, bei denen jeder Alkylteil 1 bis 4 Kohlenstoffatome enthält, dem Piperidino-, Morpholino-, 1-Piperazinylrest (gegebenenfalls in 4-Stellung mit einem Alkylrest mit 1 bis 4 Kohlenstoffatomen oder mit einem Phenylalkylrest, bei dem der Alkylteil 1 bis 4 Kohlenstoffatome enthält, substituiert), den Cycloalkylresten mit 3 bis 6 Kohlenstoffatomen, den Cycloalkenylresten mit 4 bis 6 Kohlenstoffatomen, dem Phenylrest (gegebenenfalls mit einem oder mehreren Atomen oder Resten substituiert, die unter den Halogenatomen und den Alkylresten mit 1 bis 4 Kohlenstoffatomen oder Alkoxyresten mit 1 bis 4 Kohlenstoffatomen ausgewählt sind), dem Cyanorest, dem Carboxyrest oder den Alkoxycarbonylresten, bei denen der Alkylteil 1 bis 4 Kohlenstoffatome enthält,
- einen Phenyl- oder α- oder β-Naphthylrest, der gegebenenfalls mit einem oder mehreren Atomen oder Resten substituiert ist, die unter den Halogenatomen und den Alkylresten mit 1 bis 4 Kohlenstoffatomen oder den Alkoxyresten mit 1 bis 4 Kohlenstoffatomen ausgewählt sind, oder einen aromatischen heterocyclischen Rest mit 5 Ringgliedern, der vorzugsweise unter dem Furyl- und Thienylrest ausgewählt ist,
- oder einen gesättigten heterocyclischen Rest mit 4 bis 6 Kohlenstoffatomen, der gegebenenfalls mit einem oder mehreren Alkylresten mit 1 bis 4 Kohlenstoffatomen substituiert ist,
R₃ einen
- geraden oder verzweigten Alkylrest mit 1 bis 8 Kohlenstoffatomen,
- geraden oder verzweigten Alkenylrest mit 2 bis 8 Kohlenstoffatomen,
- geraden oder verzweigten Alkinylrest mit 2 bis 8 Kohlenstoffatomen,
- Cycloalkylrest mit 3 bis 6 Kohlenstoffatomen,
- Phenyl- oder α- oder β-Naphthylrest
darstellt, der gegebenenfalls mit einem oder mehreren Atomen oder Resten substituiert ist, die ausgewählt sind unter den Halogenatomen und den Alkyl-, Alkenyl-, Alkinyl-, Aryl-, Aralkyl-, Alkoxy-, Alkylthio-, Aryloxy-, Arylthio-, Hydroxy-, Hydroxyalkyl-, Mercapto-, Formyl-, Acyl-, Acylamino-, Aroylamino-, Alkoxycarbonylamino-, Amino-, Alkylamino-, Dialkylamino-, Carboxy-, Alkoxycarbonyl-, Carbamoyl-, Alkylcarbamoyl-, Dialkylcarbamoyl-, Cyano-, Nitro- und Trifluormethylresten,
- oder einen aromatischen Heterocyclus mit 5 Ringgliedern (darstellt), der ein oder mehrere, gleiche oder verschiedene Heteroatome enthält, die unter den Stickstoff-, Sauerstoff- oder Schwefelatomen ausgewählt sind, und gegebenenfalls mit einem oder mehreren, gleichen oder verschiedenen Substituenten substituiert ist, die unter den Halogenatomen und den Alkyl-, Aryl-, Amino-, Alkylamino-, Dialkylamino-, Alkoxycarbonylamino-, Acyl-, Arylcarbonyl-, Cyano-, Carboxy-, Carbamoyl-, Alkylcarbamoyl-, Dialkylcarbamoyl- oder Alkoxycarbonylresten ausgewählt sind, wobei es sich versteht, dass die Alkylreste bei den Substituenten der Phenyl-, α- oder β-Naphthylreste und der aromatischen heterocyclischen Reste und die Alkylteile der anderen Reste 1 bis 4 Kohlenstoffatome enthalten und dass die Alkenyl- und Alkinylreste 2 bis 8 Kohlenstoffatome enthalten und dass die Arylreste Phenyl- oder α- oder β-Naphthylreste sind,
R₄ einen
- Alkoxyrest mit 1 bis 6 Kohlenstoffatomen in gerader oder verzweigter Kette,
- Alkenyloxyrest mit 3 bis 6 Kohlenstoffatomen in gerader oder verzweigter Kette,
- Alkinyloxyrest mit 3 bis 6 Kohlenstoffatomen in gerader oder verzweigter Kette,
- Cycloalkyloxyrest mit 3 bis 6 Kohlenstoffatomen,
- Cycloalkenyloxyrest mit 3 bis 6 Kohlenstoffatomen
darstellt, der gegebenenfalls substituiert ist mit einem oder mehreren Halogenatomen oder mit einem Alkoxyrest mit 1 bis 4 Kohlenstoffatomen, einem Alkylthiorest mit 1 bis 4 Kohlenstoffatomen, oder einen Carboxy-, einen Alkyloxycarbonylrest (darstellt), dessen Alkylteil 1 bis 4 Kohlenstoffatome enthält, einen Cyano-, Carbamoyl-, N-Alkylcarbamoyl- oder N,N-Dialkylcarbamoylrest (darstellt), bei dem jeder Alkylteil 1 bis 4 Kohlenstoffatome enthält oder mit dem Stickstoffatom, an das er gebunden ist, einen gesättigten heterocyclischen Rest bildet, der 5 oder 6 Ringglieder und gegebenenfalls ein zweites unter den Sauerstoff-, Schwefel- oder Stickstoffatomen ausgewähltes Heteroatom enthält (und) gegebenenfalls mit einem Alkylrest mit 1 bis 4 Kohlenstoffatomen substituiert ist, oder einen Phenylrest oder einen Phenylalkylrest (darstellt), dessen Alkylteil 1 bis 4 Kohlenstoffatome enthält.

2. Taxoide gemäß Anspruch 1, bei denen Z ein Wasserstoffatom oder einen Rest der allgemeinen Formel (II) darstellt, in der R₁ einen Benzoylrest oder einen Rest R₂-O-CO- darstellt, in dem R₂ einen tert-Butylrest darstellt, und R₃ einen Alkylrest mit 1 bis 6 Kohlenstoffatomen, Alkenylrest mit 2 bis 6 Kohlenstoffatomen, Cycloalkylrest mit 3 bis 6 Kohlenstoffatomen, Phenylrest, der gegebenenfalls mit einem oder mehreren, gleichen oder verschiedenen Atomen oder Resten substituiert ist, die unter den Halogenatomen und den Resten Alkyl, Alkoxy, Dialkylamino, Acylamino, Alkoxycarbonylamino oder Trifluormethyl ausgewählt sind, oder einen 2- oder
3-Furyl-, 2- oder 3-Thienyl- oder 2-, 4- oder 5-Thiazolylrest darstellt, und R₄ einen geraden oder verzweigten Alkyloxyrest mit 1 bis 6 Kohlenstoffatomen darstellt.

3. Taxoide gemäß Anspruch 1, bei denen Z ein Wasserstoffatom oder einen Rest der allgemeinen Formel (II) darstellt, in der R₁ einen Benzoylrest oder einen Rest R₂-O-CO- darstellt, in dem R₂ einen tert-Butylrest darstellt und R₃ einen Isobutyl-, Isobutenyl-, Butenyl-, Cyclohexyl-, Phenyl-, 2-Furyl-, 3-Furyl-, 2-Thienyl-, 3-Thienyl-, 2-Thiazolyl-, 4-Thiazolyl- oder 5-Thiazolylrest darstellt, R₄ einen Methoxy-, Ethoxy- oder Propoxyrest darstellt.

4. Verfahren zur Herstellung eines Produkts gemäß einem der Ansprüche 1, 2 oder 3, dadurch gekennzeichnet, daß man ein Produkt der allgemeinen Formel: 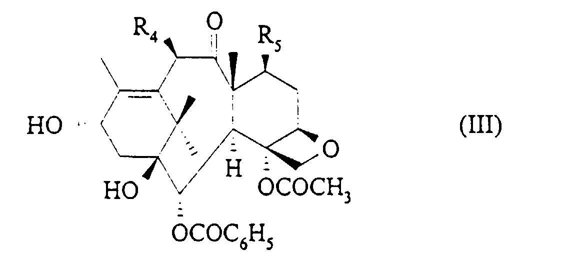 in der R₄ wie in einem der Ansprüche 1, 2 oder 3 definiert ist und R₅ einen Trifluormethansulfonyloxyrest darstellt oder eine Bindung mit dem Kohlenstoffatom des Methylrests in α-Stellung bildet, um einen Cyclopropanring zu bilden, mittels einer Säure der allgemeinen Formel: 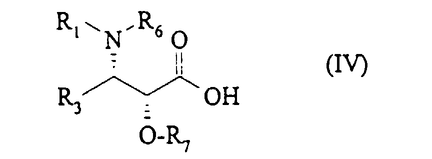 in der R₁ und R₃ wie in einem der Ansprüche 1, 2 oder 3 definiert sind oder aber R₆ ein Wasserstoffatom darstellt und R₇ eine Schutzgruppe der Hydroxyfunktion darstellt oder aber R₆ und R₇ zusammen einen gesättigten Heterocyclus mit 5 oder 6 Ringgliedern bilden, oder mittels eines Derivats dieser Säure verestert, um einen Ester der allgemeinen Formel: 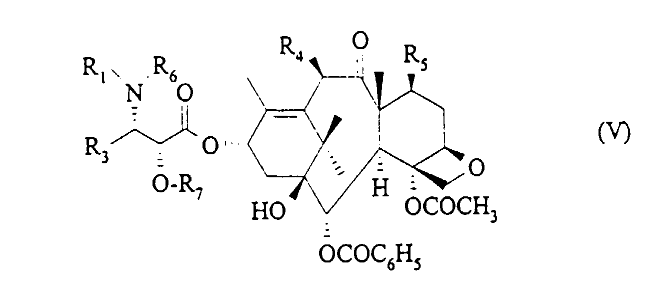 zu erhalten, in der R₁, R₃, R₄, R₅, R₆ und R₇ wie zuvor definiert sind, dann die durch R₇ und/oder R₆ und R₇ dargestellten Schutzgruppen durch Wasserstoffatome ersetzt und, wenn R₅ einen Trifluormethansulfonyloxyrest darstellt, diesen Rest entfernt, um einen Cyclopropanring mit dem Kohlenstoffatom des Methylrests in α-Stellung zu bilden.

5. Verfahren gemäß Anspruch 4, dadurch gekennzeichnet, dass man die Veresterung mittels einer Säure der allgemeinen Formel (IV) in Gegenwart eines Kondensationsmittels und eines Aktivierungsmittels in einem organischen Lösungsmittel bei einer Temperatur zwischen -10 und 90 °C ausführt.

6. Verfahren gemäß Anspruch 4, dadurch gekennzeichnet, dass man die Veresterung mittels einer Säure der allgemeinen Formel (IV) in Form des symmetrischen Anhydrids ausführt, indem man in Gegenwart eines Aktivierungsmittels in einem organischen Lösungsmittel bei einer Temperatur zwischen 0 und 90 °C arbeitet.

7. Verfahren gemäß Anspruch 4, dadurch gekennzeichnet, dass man die Veresterung unter Verwendung der Säure der allgemeinen Formel (IV) in Form des Halogenids oder in Form des gemischten Anhydrids mit einer aliphatischen oder aromatischen Säure, das gegebenenfalls in situ hergestellt wird, in Gegenwart einer Base ausführt, indem man in einem organischen Lösungsmittel bei einer Temperatur zwischen 0 und 80 °C arbeitet.

8. Verfahren gemäß Anspruch 4, dadurch gekennzeichnet, dass man die Schutzgruppen R₇ und/oder R₆ und R₇ durch Wasserstoffatome ersetzt, indem man auf die folgende Weise vorgeht:
1) wenn R₆ ein Wasserstoffatom darstellt und R₇ eine Schutzgruppe der Hydroxyfunktion darstellt, führt man das Ersetzen der Schutzgruppen durch Wasserstoffatome mittels einer Mineralsäure oder organischen Säure, die allein oder im Gemisch verwendet wird, aus, indem man in einem organischen Lösungsmittel, das unter den Alkoholen, den Ethern, den Estern, den aliphatischen Kohlenwasserstoffen, den halogenierten aliphatischen Kohlenwasserstoffen, den aromatischen Kohlenwasserstoffen oder den Nitrilen ausgewählt ist, bei einer Temperatur zwischen -10 und 60 °C arbeitet,
2) wenn R₆ und R₇ zusammen einen gesättigten Heterocyclus mit 5 oder 6 Ringgliedern und spezieller einen Oxazolidinring der allgemeinen Formel 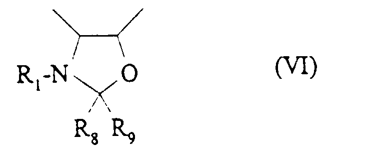 bilden, in der R₁ wie in einem der Ansprüche 1, 2 oder 3 definiert ist, R₈ und R₉, die gleich oder verschieden sind, ein Wasserstoffatom oder einen Alkylrest mit 1 bis 4 Kohlenstoffatomen oder einen Aralkylrest, dessen Alkylteil 1 bis 4 Kohlenstoffatome enthält und dessen Arylteil einen Phenylrest darstellt, der gegebenenfalls mit einem oder mehreren Alkoxyresten mit 1 bis 4 Kohlenstoffatomen substituiert ist, oder einen Arylrest darstellt, der einen Phenylrest darstellt, der gegebenenfalls mit einem oder mehreren Alkoxyresten mit 1 bis 4 Kohlenstoffatomen substituiert ist, oder aber R₈ einen Alkoxyrest mit 1 bis 4 Kohlenstoffatomen oder einen Trihalomethylrest oder einen Phenylrest, der mit einem Trihalomethylrest substituiert ist, darstellt und R₉ ein Wasserstoffatom darstellt, oder aber R₈ und R₉ zusammen mit dem Kohlenstoffatom, an das sie gebunden sind, einen Ring mit 4 bis 7 Ringgliedem bilden, ersetzt man die durch R₆ und R₇ gebildete Schutzgruppe durch Wasserstoffatome, indem man, gemäß den Bedeutungen von R₁, R₈ und R₉ auf die folgende Weise vorgeht:
a) wenn R₁ einen tert-Butoxycarbonylrest darstellt, R₈ und R₉, die gleich oder verschieden sind, einen Alkylrest oder einen Aralkyl- oder Arylrest darstellen, oder aber R₈ einen Trihalomethylrest oder einen mit einem Trihalomethylrest substituierten Phenylrest darstellt und R₉ ein Wasserstoffatom darstellt, oder aber R₈ und R₉ zusammen einen Ring mit 4 bis 7 Ringgliedern bilden, behandelt man Ester der allgemeinen Formel (V) mit einer Mineralsäure oder organischen Säure, gegebenenfalls in einem organischen Lösungsmittel, wie einem Alkohol, um ein Produkt der allgemeinen Formel: 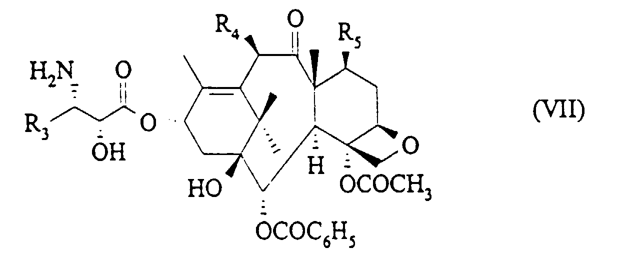 zu erhalten, in der R₃, R₄ und R₅ in einem der Ansprüche 1, 2 oder 3 definiert sind, das man mittels Benzoylchlorid, in dem der Phenylring gegebenenfalls substituiert ist, Thenoylchlorid, Furoylchlorid oder einem Produkt der allgemeinen Formel:
R₂-O-CO-X (VIII)
acyliert, in der R₂ wie zuvor definiert ist und X ein Halogenatom oder einen Rest -O-R₂ oder -O-CO-O-R₂ darstellt, um ein Produkt der allgemeinen Formel (I) zu erhalten, in der Z einen Rest der allgemeinen Formel (II) darstellt.
b) wenn R₁ einen gegebenenfalls substituierten Benzoyl-, Thenoyl- oder Furoylrest oder einen Rest R₂O-CO- darstellt, in dem R₂ wie zuvor definiert ist, R₈ ein Wasserstoffatom oder einen Alkoxyrest mit 1 bis 4 Kohlenstoffatomen oder einen Phenylrest, der mit einem oder mehreren Alkoxyresten mit 1 bis 4 Kohlenstoffatomen substituiert ist, darstellt und R₉ ein Wasserstoffatom darstellt, ersetzt man die durch R₆ und R₇ gebildete Schutzgruppe durch Wasserstoffatome in Gegenwart einer Mineralsäure oder organischen Säure, die allein oder im Gemisch in stöchiometrischer oder katalytischer Menge verwendet wird, indem man in einem organischen Lösungsmittel, das unter den Alkoholen, den Ethern, den Estern, den aliphatischen Kohlenwasserstoffen, den halogenierten aliphatischen Kohlenwasserstoffen und den aromatischen Kohlenwasserstoffen ausgewählt ist, bei einer Temperatur zwischen -10 und 60 °C, vorzugsweise zwischen 15 und 30 °C, arbeitet.

9. Verfahren gemäß Anspruch 4, dadurch gekennzeichnet, dass man den Rest R₅, der einen Trifluormethansulfonyloxyrest darstellt, mittels eines Alkalimetallhalogenids oder eines Alkalimetallazids oder eines Ammoniumsalzes entfernt, um eine Bindung mit dem Kohlenstoffatom des Methylrests in α-Stellung zu bilden, indem man in einem organischen Lösungsmittel, das unter den Ethern, den Nitrilen oder den aliphatischen Estern ausgewählt ist, allein oder im Gemisch, bei einer Temperatur zwischen 20 °C und der Siedetemperatur des Reaktionsgemischs arbeitet.

10. Verfahren zur Herstellung eines neuen Taxoids der allgemeinen Formel: 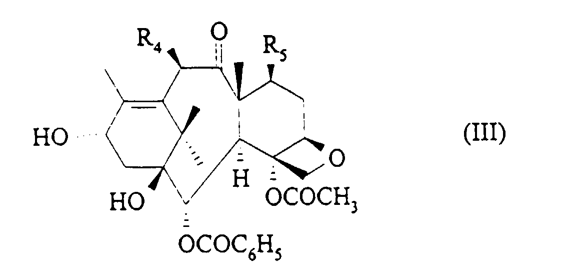 in der R₄ wie in einem der Ansprüche 1, 2 oder 3 definiert ist und R₅ einen Trifluormethansulfonyloxyrest darstellt oder eine Bindung mit dem Kohlenstoffatom des Methylrests in α-Stellung bildet, dadurch gekennzeichnet, dass man 10-Desacetylbaccatin III der Formel: 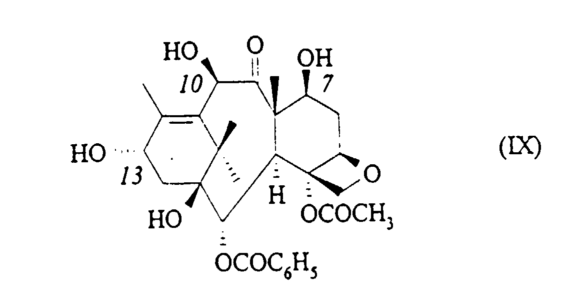 mit einem Silylhalogenid der allgemeinen Formel:
(R')₃-Si-Hal (X)
behandelt, in der die Symbole R', die gleich oder verschieden sind, einen Alkylrest mit 1 bis 4 Kohlenstoffatomen, der gegebenenfalls mit einem Phenylrest substituiert ist, oder einen Phenylrest darstellen, um ein Produkt der allgemeinen Formel: 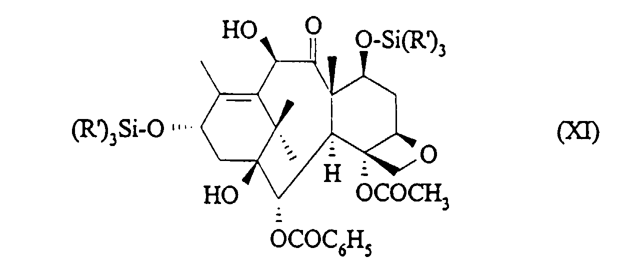 zu erhalten, in der R' wie zuvor definiert ist, das man mit einem Produkt der allgemeinen Formel:
R'₄-X₁ (XII)
behandelt, in der R'₄ so ist, dass R'₄-O identisch mit R₄ wie in einem der Ansprüche 1, 2 oder 3 ist, und X₁ ein Halogenatom oder einen reaktiven Esterrest, wie einen Schwefelsäure- oder Sulfonsäureesterrest, darstellt, um ein Produkt der allgemeinen Formel: 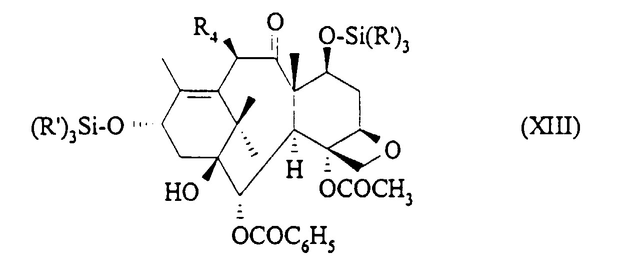 zu erhalten, in der R' und R₄ wie zuvor definiert sind, bei dem man die silylierten Schutzgruppen durch Wasserstoffatome ersetzt, um ein Produkt der allgemeinen Formel: 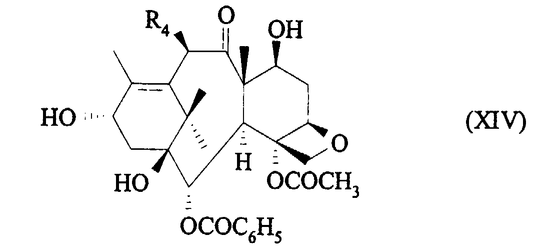 zu erhalten, in der R₄ wie zuvor definiert ist, das man mit einem Trifluormethansulfonsäurederivat behandelt, um ein Produkt der allgemeinen Formel (III) zu erhalten, in der R₅ einen Trifluormethansulfonyloxyrest darstellt, das man gegebenenfalls mit einem Alkalimetallhalogenid oder einem Alkalimetallazid oder einem Ammoniumsalz behandelt, indem man in einem organischen Lösungsmittel, das unter den Ethern, den Nitrilen und den aliphatischen Estern ausgewählt ist, allein oder im Gemisch, bei einer Temperatur zwischen 20 °C und der Siedetemperatur des Reaktionsgemischs arbeitet, um ein Produkt der allgemeinen Formel (III), in der R₅ eine Bindung mit dem Kohlenstoffatom des Methylrests in α-Stellung bildet, zu erhalten.

11. (4α-Acetoxy-2α-benzoyloxy-5β,20-epoxy-1β-hydroxy-10β-methoxy-7β,8β-methylen-9-oxo-19-nor-11-taxen-13α-yl)-(2R,3S)-3-tert-butoxycarbonylamino-2-hydroxy-3-phenyl-propionat.

12. (4α-Acetoxy-2α-benzoyloxy-5β,20-epoxy-1β-hydroxy-10β-ethoxy-7β,8β-methylen-9-oxo-19-nor-11-taxen-13α-yl)-(2R,3S)-3-tert-butoxycarbonylamino-2-hydroxy-3-phenyl-propionat.

13. (4α-Acetoxy-2α-benzoyloxy-5β,20-epoxy-1β-hydroxy-10β-(1-propyl)-oxy-7β,8β-methylen-9-oxo-19-nor-11-taxen-13α-yl)-(2R,3S)-3-tert-butoxycarbonylamino-2-hydroxy-3-phenyl-propionat.

14. Pharmazeutische Zusammensetzung, dadurch gekennzeichnet, dass sie wenigstens ein Produkt gemäß einem der Ansprüche 1, 2 oder 3, für das Z einen Rest der allgemeinen Formel (II) darstellt, in Verbindung mit einem oder mehreren Verdünnungsmitteln oder pharmazeutisch zulässigen Adjuvantien und gegebenenfalls einer oder mehreren kompatiblen und pharmakologisch aktiven Verbindung enthält.

15. Pharmazeutische Zusammensetzung, dadurch gekennzeichnet, dass sie wenigstens ein Produkt gemäß Anspruch 11 in Verbindung mit einem oder mehreren Verdünnungsmitteln oder pharmazeutisch zulässigen Adjuvantien und gegebenenfalls einer oder mehreren kompatiblen und pharmakologisch aktiven Verbindung enthält.

16. Pharmazeutische Zusammensetzung, dadurch gekennzeichnet, dass sie wenigstens ein Produkt gemäß Anspruch 12 in Verbindung mit einem oder mehreren Verdünnungsmitteln oder pharmazeutisch zulässigen Adjuvantien und gegebenenfalls einer oder mehreren kompatiblen und pharmakologisch aktiven Verbindung enthält.

17. Pharmazeutische Zusammensetzung, dadurch gekennzeichnet, dass sie wenigstens ein Produkt gemäß Anspruch 13 in Verbindung mit einem oder mehreren Verdünnungsmitteln oder pharmazeutisch zulässigen Adjuvantien und gegebenenfalls einer oder mehreren kompatiblen und pharmakologisch aktiven Verbindung enthält.

## Claims

1. Taxoids of general formula: 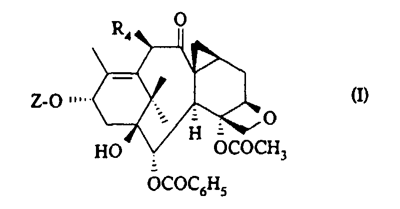 in which:
Z represents a hydrogen atom or a radical of general formula: 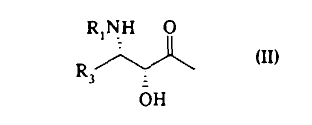 in which:
R₁ represents a benzoyl radical optionally substituted with one or more atoms or radicals, which may be identical or different, chosen from halogen atoms and alkyl radicals containing 1 to 4 carbon atoms, alkoxy radicals containing 1 to 4 carbon atoms or trifluoromethyl, thenoyl or furoyl radicals or a radical R₂-O-CO- in which R₂ represents:
- an alkyl radical containing 1 to 8 carbon atoms, an alkenyl radical containing 2 to 8 carbon atoms, an alkynyl radical containing 3 to 8 carbon atoms, a cycloalkyl radical containing 3 to 6 carbon atoms, a cycloalkenyl radical containing 4 to 6 carbon atoms or a bicycloalkyl radical containing 7 to 10 carbon atoms, these radicals optionally being substituted with one or more substituents chosen from halogen atoms and hydroxyl radicals, alkoxy radicals containing 1 to 4 carbon atoms, dialkylamino radicals in which each alkyl part contains 1 to 4 carbon atoms, piperidino, morpholino or 1-piperazinyl radicals (optionally substituted at -4 with an alkyl radical containing 1 to 4 carbon atoms or with a phenylalkyl radical in which the alkyl part contains 1 to 4 carbon atoms), cycloalkyl radicals containing 3 to 6 carbon atoms, cycloalkenyl radicals containing 4 to 6 carbon atoms, phenyl radicals (optionally substituted with one or more atoms or radicals chosen from halogen atoms and alkyl radicals containing 1 to 4 carbon atoms or alkoxy radicals containing 1 to 4 carbon atoms), cyano or carboxyl radicals or alkoxycarbonyl radicals in which the alkyl part contains 1 to 4 carbon atoms,
- a phenyl or α- or β-naphthyl radical optionally substituted with one or more atoms or radicals chosen from halogen atoms and alkyl radicals containing 1 to 4 carbon atoms or alkoxy radicals containing 1 to 4 carbon atoms or a 5-membered aromatic heterocyclic radical preferably chosen from furyl and thienyl radicals,
- or a saturated heterocyclic radical containing 4 to 6 carbon atoms optionally substituted with one or more alkyl radicals containing 1 to 4 carbon atoms,
R₃ represents
- a straight or branched alkyl radical containing 1 to 8 carbon atoms,
- a straight or branched alkenyl radical containing 2 to 8 carbon atoms,
- a straight or branched alkynyl radical containing 2 to 8 carbon atoms,
- a cycloalkyl radical containing 3 to 6 carbon atoms,
- a phenyl or α- or β-naphthyl radical
optionally substituted with one or more atoms or radicals chosen from halogen atoms and alkyl, alkenyl, alkynyl, aryl, aralkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, alkylcarbamoyl, dialkylcarbamoyl, cyano, nitro and trifluoromethyl radicals,
- or a 5-membered aromatic heterocycle containing one or more hetero atoms, which may be identical or different, chosen from nitrogen, oxygen or sulphur atoms and optionally substituted with one or more substituents, which may be identical or different, chosen from halogen atoms and alkyl, aryl, amino, alkylamino, dialkylamino, alkoxycarbonylamino, acyl, arylcarbonyl, cyano, carboxyl, carbamoyl, alkylcarbamoyl, dialkylcarbamoyl or alkoxycarbonyl radicals, it being understood that, in the substituents of the phenyl, α- or β-naphthyl radicals and aromatic heterocyclic radicals, the alkyl radicals and the alkyl portions of the other radicals contain 1 to 4 carbon atoms and that the alkenyl and alkynyl radicals contain 2 to 8 carbon atoms and that the aryl radicals are phenyl or α- or β-naphthyl radicals.
R₄ represents
- an alkoxy radical containing 1 to 6 carbon atoms in a straight or branched chain,
- an alkenyloxy radical containing 3 to 6 carbon atoms in a straight or branched chain,
- an alkynyloxy radical containing 3 to 6 carbon atoms in a straight or branched chain,
- a cycloalkyloxy radical containing 3 to 6 carbon atoms,
- a cycloalkenyloxy radical containing 3 to 6 carbon atoms
optionally substituted with one or more halogen atoms or with an alkoxy radical containing 1 to 4 carbon atoms, an alkylthio radical containing 1 to 4 carbon atoms or a carboxyl radical, an alkyloxycarbonyl radical in which the alkyl part contains 1 to 4 carbon atoms, a cyano or carbamoyl radical, an N-alkylcarbamoyl or N,N-dialkylcarbamoyl radical in which each alkyl part contains 1 to 4 carbon atoms or forms, with the nitrogen atom to which it is attached, a 5- or 6-membered saturated heterocyclic radical optionally containing a second hetero atom chosen from oxygen, sulphur or nitrogen atoms optionally substituted with an alkyl radical containing 1 to 4 carbon atoms or a phenyl radical or a phenylalkyl radical in which the alkyl part contains 1 to 4 carbon atoms.

2. Taxoids according to claim 1 for which Z represents a hydrogen atom or a radical of general formula (II) in which R₁ represents a benzoyl radical or a radical R₂-O-CO- in which R₂ represents a tert-butyl radical and R₃ represents an alkyl radical containing 1 to 6 carbon atoms, an alkenyl radical containing 2 to 6 carbon atoms, a cycloalkyl radical containing 3 to 6 carbon atoms, a phenyl radical optionally substituted with one or more atoms or radicals, which may be identical or different, chosen from halogen atoms and alkyl, alkoxy, dialkylamino, acylamino, alkoxycarbonylamino or trifluoromethyl radicals or a 2- or 3-furyl or 2- or 3-thienyl or 2-, 4- or 5-thiazolyl radical and R₄ represents a straight or branched alkyloxy radical containing 1 to 6 carbon atoms.

3. Taxoids according to claim 1 for which Z represents a hydrogen atom or a radical of general formula (II) in which R₁ represents a benzoyl radical or a radical R₂-O-CO- in which R₂ represents a tert-butyl radical and R₃ represents an isobutyl, isobutenyl, butenyl, cyclohexyl, phenyl, 2-furyl, 3-furyl, 2-thienyl, 3-thienyl, 2-thiazolyl, 4-thiazolyl or 5-thiazolyl radical and R₄ represents a methoxy, ethoxy or propoxy radical.

4. Process for the preparation of a product according to one of claims 1, 2 or 3, characterized in that a product of general formula: 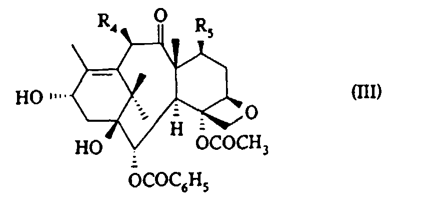 in which R₄ is defined as in one of claims 1, 2 or 3 and R₅ represents a trifluoromethanesulphonyloxy radical or forms a bond with the carbon atom of the α-methyl radical, is esterified so as to form a cyclopropane ring, using an acid of general formula: 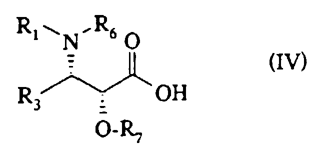 in which R₁ and R₃ are defined as in one of claims 1, 2 or 3, or alteratively R₆ represents a hydrogen atom and R₇ represents a protecting group for the hydroxyl function, and either R₆ and R₇ together form a 5- or 6-membered saturated heterocycle, or a derivative of this acid is esterified, in order to obtain an ester of general formula: 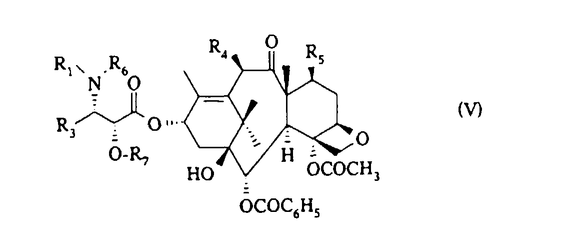 in which R₁, R₃, R₄, R₅, R₆ and R₇ are defined as above, then the protecting groups represented by R₇ and/or R₆ and R₇ are replaced by hydrogen atoms and, when R₅ represents a trifluoromethanesulphonyloxy radical, this radical is removed so as to form a cyclopropane ring with the carbon atom of the α-methyl radical.

5. Process according to claim 4, characterized in that the esterification is carried out using an acid of general formula (IV) in the presence of a coupling agent and an activating agent in an organic solvent at a temperature of between -10 and 90°C.

6. Process according to claim 4, characterized in that the esterification is carried out using an acid of general formula (IV) in symmetrical anhydride form, working in the presence of an activating agent in an organic solvent at a temperature of between 0 and 90°C.

7. Process according to claim 4, characterized in that the esterification is carried out using the acid of general formula (IV) in the form of the halide or in the form of the mixed anhydride with an aliphatic or aromatic acid, optionally prepared in situ, in the presence of a base, working in an organic solvent at a temperature of between 0 and 80°C.

8. Process according to claim 4, characterized in that the protecting groups R₇ and/or R₆ and R₇ are replaced by hydrogen atoms, working in the following manner:
1) when R₆ represents a hydrogen atom and R₇ represents a protecting group for the hydroxyl function, the protecting groups are replaced by hydrogen atoms using an inorganic acid or organic acid used alone or as a mixture, working in an organic solvent chosen from alcohols, ethers, esters, aliphatic hydrocarbons, halogenated aliphatic hydrocarbons, aromatic hydrocarbons or nitriles, at a temperature of between -10 and 60°C,
2) when R₆ and R₇ together form a 5- or 6-membered heterocycle and more particularly an oxazolidine ring of general formula: 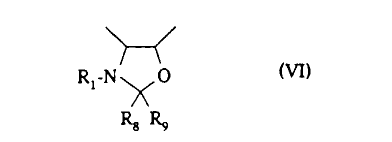 in which R₁ is defined as in one of claims 1, 2 or 3, R₈ and R₉, which may be identical or different, represent a hydrogen atom or an alkyl radical containing 1 to 4 carbon atoms or an aralkyl radical in which the alkyl part contains 1 to 4 carbon atoms and the aryl part represents a phenyl radical optionally substituted with one or more alkoxy radicals containing 1 to 4 carbon atoms, or an aryl radical representing a phenyl radical optionally substituted with one or more alkoxy radicals containing 1 to 4 carbon atoms, or alternatively R₈ represents an alkoxy radical containing 1 to 4 carbon atoms or a trihalomethyl radical or a phenyl radical substituted with a trihalomethyl radical and R₉ represents a hydrogen atom, or alternatively R₈ and R₉ form, together with the carbon atom to which they are attached, a 4- to 7-membered ring, the protecting group formed by R₆ and R₇ is replaced by hydrogen atoms, working, depending on the meanings of R₁, R₈ and R₉, in the following way:
a) when R₁ represents a tert-butoxycarbonyl radical, R₈ and R₉, which may be identical or different, represent an alkyl radical or an aralkyl or aryl radical, or alternatively R₈ represents a trihalomethyl radical or a phenyl radical substituted with a trihalomethyl radical, and R₉ represents a hydrogen atom, or alternatively R₈ and R₉ together form a 4- to 7-membered ring, the ester of general formula (V) is treated with an inorganic or organic acid, optionally in an organic solvent such as an alcohol, to give a product of general formula: 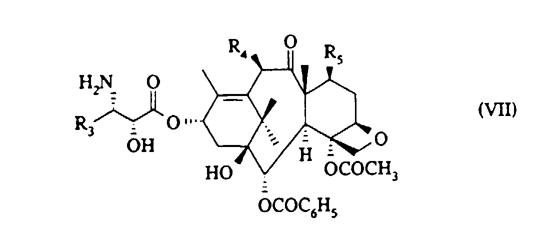 in which R₃, R₄ and R₅ are defined as in one of claims 1, 2 or 3, which product is acylated using benzoyl chloride in which the phenyl ring is optionally substituted, thenoyl chloride, furoyl chloride or a product of general formula:
R₂-O-CO-X (VIII),
in which R₂ is defined as above and X represents a halogen atom or a residue -O-R₂ or -O-CO-O-R₂, in order to obtain a product of general formula (I) in which Z represents a radical of general formula (II),
b) when R₁ represents an optionally substituted benzoyl radical, a thenoyl or furoyl radical or a radical R₂O-CO- in which R₂ is defined as above, R₈ represents a hydrogen atom or an alkoxy radical containing 1 to 4 carbon atoms or a phenyl radical substituted with one or more alkoxy radicals containing 1 to 4 carbon atoms and R₉ represents a hydrogen atom, the protecting group formed by R₆ and R₇ is replaced by hydrogen atoms in the presence of an inorganic acid or an organic acid used alone or as a mixture, in stoichiometric or catalytic amount, working in an organic solvent chosen from alcohols, ethers, esters, aliphatic hydrocarbons, halogenated aliphatic hydrocarbons and aromatic hydrocarbons, at a temperature of between -10 and 60°C, preferably between 15 and 30°C.

9. Process according to claim 4, characterized in that the radical R₅ representing a trifluoromethanesulphonyloxy radical is eliminated in order to form a bond with the carbon atom of the α-methyl radical using an alkaline metal halide or an alkaline metal azide or an ammonium salt, working in an organic solvent chosen from ethers, nitriles or aliphatic esters, alone or as a mixture, at a temperature of between 20°C and the boiling point of the reaction mixture.

10. Process for the preparation of a novel taxoid of general formula: 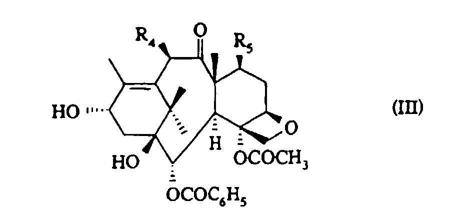 in which R₄ is defined as in one of claims 1, 2 or 3 and R₅ represents a trifluoromethanesulphonyloxy radical or forms a bond with the carbon atom of the α-methyl radical, characterized in that 10-deacetylbaccatin III of formula: 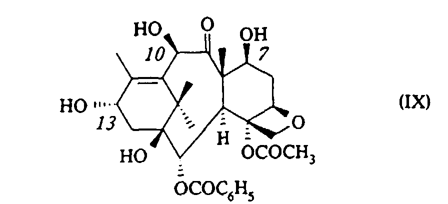 is treated with a silyl halide of general formula:
(R')₃-Si-Hal (X)
in which the symbols R', which may be identical or different, represent an alkyl radical containing 1 to 4 carbon atoms optionally substituted with a phenyl radical, or a phenyl radical, in order to obtain a product of general formula: 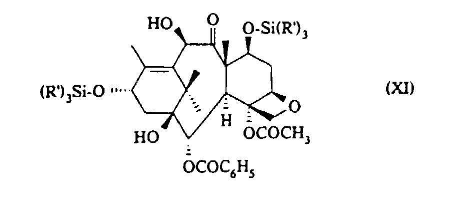 in which R' is defined as above, which product is treated with a product of general formula:
R'₄-X₁ (XII)
in which R'₄ is such that R'₄-O is identical to R₄ as in one of claims 1, 2 or 3 and X₁ represents a halogen atom or a reactive ester residue such as a sulphuric or sulphonic ester residue, in order to obtain a product of general formula: 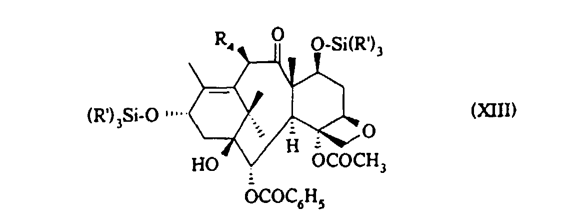 in which R' and R₄ are defined as above, the silyl protecting groups of which are replaced by hydrogen atoms in order to obtain a product of general formula: 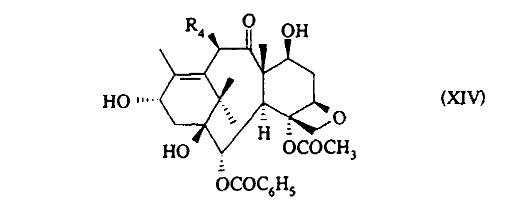 in which R₄ is defined as above, which product is treated with a trifluoromethanesulphonic acid derivative in order to obtain a product of general formula (III) in which R₅ represents a trifluoromethanesulphonyloxy radical, which product is optionally treated with an alkali metal halide or an alkali metal azide or an ammonium salt, working in an organic solvent chosen from ethers and nitriles and aliphatic esters, alone or as a mixture, at a temperature of between 20°C and the boiling point of the reaction mixture, in order to obtain a product of general formula (III) in which R₅ forms a bond with the carbon atom of the α-methyl radical.

11. 4α-Acetoxy-2α-benzoyloxy-5β,20-epoxy-1β-hydroxy-10β-methoxy-7β,8β-methylene-9-oxo-19-nor-11-taxen-13α-yl (2R,3S)-3-tert-butoxycarbonylamino-2-hydroxy-3-phenylpropionate.

12. 4α-Acetoxy-2α-benzoyloxy-5β,20-epoxy-1β-hydroxy-10β-ethoxy-7β,8β-methylene-9-oxo-19-nor-11-taxen-13α-yl (2R,3S)-3-tert-butoxycarbonylamino-2-hydroxy-3-phenylpropionate.

13. 4α-Acetoxy-2α-benzoyloxy-5β,20-epoxy-1β-hydroxy-10β-(1-propyl)oxy-7β,8β-methylene-9-oxo-19-nor-11-taxen-13α-yl (2R,3S)-3-tert-butoxycarbonylamino-2-hydroxy-3-phenylpropionate.

14. Pharmaceutical composition, characterized in that it contains at least one product according to one of claims 1, 2 or 3 for which Z represents a radical of general formula (II), in combination with one or more pharmaceutically acceptable diluents or adjuvants and optionally one or more compatible and pharmacologically active compounds.

15. Pharmaceutical composition, characterized in that it contains at least one product according to claim 11, in combination with one or more pharmaceutically acceptable diluents or adjuvants and optionally one or more compatible and pharmacologically active compounds.

16. Pharmaceutical composition, characterized in that it contains at least one product according to claim 12, in combination with one or more pharmaceutically acceptable diluents or adjuvants and optionally one or more compatible and pharmacologically active compounds.

17. Pharmaceutical composition, characterized in that it contains at least one product according to claim 13, in combination with one or more pharmaceutically acceptable diluents or adjuvants and optionally one or more compatible and pharmacologically active compounds.
